# EUROPEAN PATENT SPECIFICATION

(11) **EP 2 935 190 B1**
(45) Date of publication and mention of the grant of the patent: **19.09.2018**
(21) Application number: 13828835.2
(22) Date of filing: 18.12.2013
(51) Int. Cl.: C07C 45/62, C07C 29/17, C07C 29/42, C07C 33/03, C07C 33/042, C07C 41/56, C07C 43/313, C07C 45/51, C07C 45/67, C07C 49/04, C07D 311/72, C07D 319/06, B01J 27/19

(54) **(6R,10R)-6,10,14-TRIMETYLPENTADECAN-2-ONE PREPARED FROM 6,10-DIMETYLUNDECA-3,5,9-TRIEN-2-ONE**
(6R,10R)-6,10,14-TRIMETHYLPENTADECAN-2-ON, HERGESTELLT AUS 6,10-DIMETHYLUNDECA-3,5,9-TRIEN-2-ON
(6R,10R) -6,10,14-TRIMÉTHYLPENTADÉCAN-2-ONE PRÉPARÉ À PARTIR DE 6,10-DIMÉTHYLUNDÉCA-3,5,9-TRIÉN-2-ONE

(30) Priority: 18.12.2012 EP 12197846
(43) Date of publication of application: 28.10.2015
(73) Proprietor: DSM IP Assets B.V., 6411 TE Heerlen (NL)
(72) Inventor: BONRATH, Werner, 4303 Kaiseraugst (CH); MEDLOCK, Jonathan, Alan, 4303 Kaiseraugst (CH); STEMMLER, René, Tobias, 4303 Kaiseraugst (CH); VERZIJL, Gerardus, Karel, Maria, 4303 Kaiseraugst (CH); VRIES DE, Andreas, Hendrikus, Maria, 4303 Kaisraugst (CH)
(74) Representative: Dux, Roland
(86) International application number: PCT/IB2013/061084
(87) International publication number: WO 2014/097173

(56) References cited:
- WO-A1-2005/121115
- WO-A1-2006/066863
- US-A- 4 292 459
- US-A- 4 310 705
- US-A- 5 600 015
- F. GOTTWALT FISCHER ET AL: "Die Synthese des Phytols", JUSTUS LIEBIGS ANNALEN DER CHEMIE, vol. 475, 1 January 1929 (1929-01-01), pages 183-204, XP055062572, DOI: 10.1002/jlac.19294750115
- DATABASE CA [Online] CHEMICAL ABSTRACTS SERVICE, COLUMBUS, OHIO, US; IKEMOTO, TAKESHI ET AL: "Preparation of 4,6-O-alkylidene-.alpha.,.alpha.-trehalose as surfactants", XP002697173, retrieved from STN Database accession no. 1997:191855 -& JP 9 003087 A (KANEBO LTD, JAPAN) 7 January 1997 (1997-01-07)
- OSHIMA M ET AL: "Palladium-Catalyzed selective hydrogenolysis of alkenyloxiranes with formic acid", JOURNAL OF THE AMERICAN CHEMICAL SOCIETY, THE SOCIETY, vol. 111, 1 January 1989 (1989-01-01), pages 6280-6287, XP002209651, ISSN: 0002-7863, DOI: 10.1021/JA00198A045

## Description

### Technical Field

The present invention relates to the field of (6R,10R)-6,10,14-trimethyl-pentadecan-2-one and the reaction products thereof.

### Background of the invention

(6R,10R)-6,10,14-Trimethylpentadecan-2-one is an important intermediate, particularly for the synthesis of (R,R)-isophytol [= (3RS,7R,11R)-3,7,11,15-tetramethylhexadec-1-en-3-ol], (R,R)-phytol and tocopherols.

Isophytol, phytol and tocopherols are chiral substances, the latter two of which occur in nature in the form of the "all-R" stereoisomer. Phytol contains 2 stereocentres and in addition a trisubstituted carbon-carbon double bond which gives rise to E/Z-steroisomers, while isophytol and tocopherols have 3 stereocentres. Therefore, there are multiple isomers.

It has been shown that of the naturally occurring stereoisomers of tocopherols, (2R,4'R,8'R)-tocopherols, particularly (2R,4'R,8'R)-α-tocopherol, have the highest bioactivity (biopotency).

As natural sources of (2R,4'R,8'R)-tocopherols and (R,R)-phytol, however, are very limited, the market has a strong need for an effective synthesis of (2R,4'R,8'R)-tocopherols and (R,R)-isophytol and (6R,10R)-6,10,14-trimethylpentadecan-2-one, the starting material of these products, which is useful for industrial scale application.

As, furthermore, higher bioactivity (biopotency) has been shown, for example by H. Weiser et al. in J. Nutr. 1996, 126(10), 2539-49, to occur in general by tocopherols having the R-configuration at the chiral centre situated next to the ether oxygen atom in the ring of the molecule (i.e. 2R-configuration), as compared to the corresponding isomers having S-configuration, there is a strong need for an effective and industrial scale synthesis of (2R,4'R,8'R)-tocopherols, particularly (2R,4'R,8'R)-alpha-tocopherol.

Fischer et al., Justus Liebigs Annalen der Chemie 475 (1929), 183-204, shows a process for manufacturing 6,10,14-trimethylpentadecan-2-one wherein 6,10-dimethylundeca-3,5,9-trien-2-one is hydrogenated to 6,10-dimethylundecan-2-one, 6,10-dimethylundecan-2-one is chemically transformed to 6,10,14-trimethylpentadec-5-en-2-one, and 6,10,14-trimethylpentadec-5-en-2-one is hydrogenated to 6,10,14-trimethylpentadecan-2-one.

### Summary of the invention

Therefore, the problem to be solved by the present invention is to offer a process for the manufacturing (6R,10R)-6,10,14-trimethylpentadecan-2-one.

Surprisingly, it has been found that the process according to claim 1 is able to solve this problem. It has been shown that it is possible to obtain one specific isomer of interest from a mixture of isomers of starting material, i. e. a mixture of ((3E,5E)-/ (3E,5Z)-/ (3Z,5E)-/ and (3Z,5Z)-6,10-dimethylundeca-3,5,9-trien-2-one.

Preferred embodiments of the inventions allow making use of the non-desired isomers, by using a cis/trans-isomerization. The asymmetric hydrogenation, which is one of the key elements of this invention, can be improved in quality and speed by ketalization of the ketones to be asymmetrically hydrogenated as well as by the use of specific additives.

The process of the invention allows the production of the target molecules efficiently in a high quality from isomeric mixtures, allowing it to be used for industrial scale production. The process is very advantageous in that it forms the desired chiral product from a mixture of stereoisomers of the starting product in an efficient way.

Further aspects of the invention are subject of further independent claims. Particularly preferred embodiments are subject of dependent claims.

### Detailed description of the invention

In a first aspect the present invention relates to a process of manufacturing (6R,10R)-6,10,14-trimethylpentadecan-2-one in a multistep synthesis from 6,10-dimethylundeca-3,5,9-trien-2-one comprising the steps
a) providing a mixture of (3E,5E)-/ (3E,5Z)-/ (3Z,5E)-/ and (3Z,5Z)-6,10-dimethylundeca-3,5,9-trien-2-one;
b) separating at least one (5E)- and/or at least one (5Z)-isomers of 6,10-dimethylundeca-3,5,9-trien-2-one from the mixture of step a);
c) asymmetric hydrogenation using molecular hydrogen in the presence of a chiral iridium complex and yielding (R)-6,10-dimethylundecan-2-one ;
d) chemically transforming (R)-6,10-dimethylundecan-2-one to a mixture of (R,E)-6,10,14-trimethylpentadec-5-en-2-one and (R,Z)-6,10,14-trimethyl-pentadec-5-en-2-one ;
e) separating one isomer of 6,10,14-trimethylpentadec-5-en-2-one from the mixture obtained in step d);
f) asymmetric hydrogenation using molecular hydrogen in the presence of a chiral iridium complex and yielding (6R,10R)-6,10,14-trimethylpentadecan-2-one ;
wherein the steps a)-f) are in the order a,b,c,d,e,f.

The term "independently from each other" in this document means, in the context of substituents, moieties, or groups, that identically designated substituents, moieties, or groups can occur simultaneously with a different meaning in the same molecule.

A "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

A "C_{x-y}-alkylene" group is an alkylene group comprising x to y carbon atoms, i.e., for example C₂₋C₆ alkylene group is an alkyl group comprising 2 to 6 carbon atoms. The alkylene group can be linear or branched. For example the group -CH(CH₃)-CH₂- is considered as a C₃-alkylene group.

A "phenolic alcohol" means in this document an alcohol which has a hydroxyl group which is bound directly to an aromatic group.

The term "(R,R)-isophytol" used in this document means (3RS,7R,11R)-3,7,11,15-tetramethylhexadec-1-en-3-ol).

The term "(R,R)-phytol" used in this document means (2E,7R,11R)-3.7,11,15-tetramethyl-2-hexadecen-1-ol).

Substance names starting with "poly" such as polythiol as used in the present document refer to substances formally containing two or more of the corresponding functional groups per molecule.

The term "stereogenic centre" as used in this document is an atom, bearing groups such that interchanging of any two of the groups leads to a stereoisomer. Stereoisomers are isomeric molecules that have the same molecular formula and sequence of bonded atoms (constitution), but that differ in the three-dimensional orientations of their atoms in space.

The configuration at a stereogenic centre is defined to be either R or S. The R/S-concept and rules for the determination of the absolute configuration in stereochemistry is known to the person skilled in the art.

In the present document a carbon-carbon double bond is defined as being "prochiral" if addition of molecular hydrogen to said carbon-carbon double bond leads to the formation of a stereogenic carbon centre.

Cis/trans isomers are configurational isomers having different orientation at the double bond. In this document the term "cis" is equivalently used for "Z" and vice versa as well as "trans" for "E" and vice versa. Therefore, for example the term "cis/trans isomerization catalyst" is equivalent to the term "E/Z isomerization catalyst".

A "cis/trans isomerization catalyst" is a catalyst which is able to isomerize a cis isomer (Z-isomer) to a cis/trans isomer mixture (E/Z isomer mixture) or to isomerize a trans isomer (E-isomer) to a cis/trans isomer (E/Z isomer mixture).

The terms "E/Z", "cis/trans" and "R/S" denote mixtures of E and Z, of cis and trans, and of R and S, respectively.

In case identical labels for symbols or groups are present in several formulae, in the present document, the definition of said group or symbol made in the context of one specific formula applies also to other formulae which comprises said same label.

In the present document any single dotted line represents the bond by which a substituent is bound to the rest of a molecule.

6,10-dimethylundeca-3,5,9-trien-2-one is a known substance and is commercially available and is a mixture of (3E,5E)-/ (3E,5Z)-/ (3Z,5E)-/ and (3Z,5Z)-6,10-dimethylundeca-3,5,9-trien-2-one. The isomers (3E,5E)- and (3E,5Z)-6,10-dimethylundeca-3,5,9-trien-2-one are the predominant ones independently from their synthesis and typically sum up together to about 95% of the weight of all isomers.

In a first step (step a) of the process a mixture (3E,5E)-/ (3E,5Z)-/ (3Z,5E)-/ and (3Z,5Z)-6,10-dimethylundeca-3,5,9-trien-2-one is provided.

### Separation

Steps b) and e) are separation steps. Step b) relates to the separation of at least one (5E)- and/or at least one (5Z)-isomers of 6,10-dimethylundeca-3,5,9-trien-2-one from the mixture of step a) and step e) relates to the separation of one of the isomers of (R)-6,10,14-trimethylpentadec-5-en-2-one from the mixture obtained in step d), respectively.

This separation of isomers in step b) and/or e) can be done in different ways. A first possibility is the separation by means of chromatography. A further and preferred way of separation is that the separation of isomers in step b) and/or e) is done by distillation. The separation is possible by the fact that the isomers have different boiling points. In order to minimize thermal degradation of the isomers it is advisable to distil under reduced pressure and by means of a distillation column.

As the isomers to be separated have different boiling points (see table 1) the isomers can be separated by distillation. Using specific distillation techniques and equipment it is possible to separate the isomer having the higher or the lower boiling point.

| **Substance** | **Boiling point** |
|---|---|
| (3E,5E)-6,10-dimethylundeca-3,5,9-trien-2-one | 100-105°C at 0.6 Torr |
| (3E,5Z)-6,10-dimethylundeca-3,5,9-trien-2-one | 92-98°C at 0.8 Torr |
| (R,E)-6,10,14-trimethylpentadec-5-en-2-one | 122 °C at 2 mbar |
| (R,Z)-6,10,14-trimethylpentadec-5-en-2-one | 119 °C at 2 mbar |

| | |
|---|---|
| Table 1. Boiling points of isomers. | |

It is important to realize that the double bond at the 3 position is not a prochiral double bond in the sense of the present inventions as upon hydrogenation it does not form a stereogenic carbon centre at the 3 or 4 position. Hence, for the separation it is only relevant that the (5E)- and/or the (5Z)-isomers can be separated as good as possible.

### Cis/trans isomerization

In a preferred embodiment the distillation is done in the presence of a cis/trans isomerization catalyst.

Cis/trans isomerization catalysts are catalysts which isomerize the carbon carbon double bonds. It has been found that for the purpose of this invention said cis/trans isomerization catalysing the cis/trans isomerization of the double bonds in the 5 and 9 position is particularly nitrogen monoxide (NO) or an organic sulphur compound, particularly a polythiol.

Particularly suitable as cis/trans isomerization catalysts are polythiols of formula (X) or aromatic polythiols 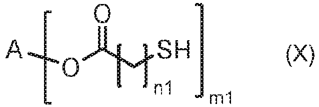 wherein n1 represents an integer from 1 to 4, particularly 2, and m1 represents an integer from 2 to 8, particularly 3 or 4, preferably 4; and A represents an aliphatic m1-valent hydrocarbon group of the molecular weight of between 28 g/mol and 400 g/mol, particularly between 90 and 150 g/mol.

The polythiols pentaerythritol tetra(mercaptoacetate), trimethylolpropane tris(mercaptoacetate), glycol dimercaptoacetate, pentaerythritol tetra-(3-mercaptopropionate), trimethylolpropanetri-(3-mercaptopropionate)(= 2-ethyl-2-(((3-mercaptopropanoyl)oxy)methyl)propane-1,3-diyl bis(3-mercaptopropanoate)) and glycol di-(3-mercaptopropionate) have been shown to be highly preferred polythiols of formula (X) and are the preferred polythiols of all the above mentioned polythiols.

Particularly preferred as aromatic polythiols are 4,4'-dimercaptobiphenyl or 4,4'-thiodibenzenethiol.

The use of polythiols of formula (X) as cis/trans isomerization catalysts is very advantageous in that polythiols have generally very low vapor pressures (i.e. high boiling points) allowing them to be used at elevated temperatures, e.g. while distilling the low boiling isomer. Furthermore, the polythiols bear a high density of thiol-functionalities per molecular weight, which is very advantageous, in that only little catalyst needs to be added.

The use of polythiol as cis/trans isomerization catalysts is very advantageous as they allow a very fast isomerization.

Nitrogen monoxide (NO) is a gas and can be introduced to the ketone or ketal to be isomerized as such or in the form of a gas mixture, particularly in combination with at least one inert gas, particularly with nitrogen. In the case a gas mixture is used the amount of nitrogen monoxide in the gas mixture is preferably in the range of 1 - 99 %, particularly of 5 -95 %, by weight of the gas mixture. Particularly, in view of corrosion and toxicity, the amount of nitrogen monoxide in the gas mixture is preferably in the range of 10 - 60 % by weight of the gas mixture.

The use of nitrogen monoxide as cis/trans isomerization catalysts is very advantageous in that the isomerization catalyst can be removed very easily from the ketone or ketal to be isomerized.

Nitrogen monoxide is preferably introduced to the ketone or ketal at atmospheric pressure or up to 1 MPa over-pressure. The over-pressure preferably amounts to 10 to 300 kPa.

Nitrogen monoxide (NO) or a mixture of nitrogen monoxide (NO) with other gases is preferably introduced in a continuous way by means of a tube and bubbled through the ketone or ketal to be isomerized.

The use of cis/trans isomerization allows the transformation of a pure cis or trans isomer or any mixtures of the isomers to yield a thermodynamically equilibrated mixture of the cis and trans isomer. Overall, this enables the separation of the desired isomer by distillation and transformation (isomerization) of the non-preferred isomer (residual isomer) into the desired isomer.

The distillation can be performed in the presence of the cis/trans isomerization catalyst (*one-pot isomerization* or *in-situ isomerization*), so that the desired isomer is re-formed continuously and can be separated by distillation.

Furthermore, the cis/trans isomerization can occur in a separate vessel in which the cis/trans isomerization catalyst is added to the remainder of the distillation. Hence, the residual isomer is isomerized by means of a cis/trans isomerization catalyst and subsequently added to the corresponding mixture of isomers provided in steps a) and d), respectively.

The use of the cis/trans isomerization in step b) and/or e) allows a high yield in the desired isomer. In preferred cases, it can be achieved that essentially all of the undesired isomer is overall isomerized to the desired isomer.

Preferably, particularly in the case where the isomerization catalyst is not nitrogen monoxide, more preferably in the case of polythiols as isomerization catalysts, the isomerization is undertaken at temperatures higher than 20° C, particularly at a temperature of between 20 °C and the boiling point of the desired isomer, particularly between 50 °C and the boiling point of the desired isomer. The isomerization can occur at ambient pressure or at reduced pressure. In case of the *one-pot isomerization* the isomerization is preferably undertaken under reduced pressure.

Particularly for the case of nitrogen monoxide being cis/trans isomerization catalyst the isomerization is undertaken at ambient or over-pressure.

It further has been observed that in the isomerization with polythiols addition of polar solvents such as amides, pyrrolidones, sulfones, sulfoxides, ionic liquids, particularly N,N-dimethylformide (DMF) or N-methyl-2-pyrrolidone (NMP), sulfolane, dimethylsulfoxide (DMSO) and 1-butyl-3-methylimidazolium bromide has an accelarating effect on the isomerization .

Therefore, it is preferred that the process of a cis/trans isomerization is undertaken in the presence of a polar solvent, particularly a polar solvent which is selected from the group consisting of ionic liquids, particularly 1-butyl-3-methylimidazolium bromide, N,N-dimethylformide (DMF), N-methyl-2-pyrrolidone (NMP), sulfolane and dimethylsulfoxide (DMSO).

More preferred it is that the process of a cis/trans isomerization is undertaken in the presence of a polar solvent, particularly a polar solvent which is selected from the group consisting of ionic liquids, particularly 1-butyl-3-methylimidazolium bromide. N,N-dimethylformide (DMF), N-methyl-2-pyrrolidone (NMP) and dimethylsulfoxide (DMSO).

The amount of cis/trans isomerization catalyst is preferably between 1 and 20 % by weight in relation to the amount of the isomers of 6,10-dimethylundeca-3,5,9-trien-2-one or to (R)-6,10,14-trimethylpentadec-5-en-2-one.

### Ketal formation

In a further embodiment before the step c) a step cₒ) takes place cₒ) forming a ketal of the isomer of 6,10-dimethylundeca-3,5,9-trien-2-one separated in step b).

Hence, in step c) the ketal of 6,10-dimethylundeca-3,5,9-trien-2-one is asymmetrically hydrogenated and after the asymmetric hydrogenation the hydrogenated ketal is hydrolysed to the ketone and yielding (R)-6,10-dimethylundecan-2-one.

In a further embodiment before the step f) a step fₒ) takes place fₒ) forming a ketal of the isomer of (R)-6,10,14-trimethylpentadec-5-en-2-one separated in step e)

Hence, in step f) the ketal of (R)-6,10,14-trimethylpentadec-5-en-2-one is asymmetrically hydrogenated and after the asymmetric hydrogenation the hydrogenated ketal is hydrolysed to the ketone and yielding (6R,10R)-6,10,14-trimethylpentadecan-2-one.

The formation of a ketal from a ketone, per se, is known to the person skilled in the art.

The ketal of an unsaturated ketone can be preferably formed from the above mentioned unsaturated ketone and an alcohol.

It is known to the person skilled in the art that there are alternative routes of synthesis for ketals. In principle, the ketal can also be formed by treating a ketone with ortho-esters or by trans-ketalization such as disclosed for example in Pério et al., Tetrahedron Letters 1997, 38 (45). 7867-7870, or in Lorette and Howard, J. Org. Chem. 1960, 521-525.

Preferably the ketal is formed from the above mentioned unsaturated ketone and an alcohol.

The alcohol can comprise one or more hydroxyl groups. The alcohol may be a phenolic alcohol or an aliphatic or cycloaliphatic alcohol. Preferably the alcohol has one or two hydroxyl groups.

In case the alcohol has one hydroxyl group, the alcohol is preferably an alcohol which has 1 to 12 carbon atoms. Particularly, the alcohol having one hydroxyl group is selected from the group consisting of methanol, ethanol, 1-propanol, 2-propanol, 1-butanol, 2-methyl-1-propanol, 2-butanol, pentane-1-ol, 3-methylbutane-1-ol, 2-methylbutane-1-ol, 2,2-dimethylpropan-1-ol, pentane-3-ol, pentane-2-ol, 3-methylbutane-2-ol, 2-methylbutan-2-ol, hexane-1-ol, hexane-2-ol, hexane-3-ol, 2-methyl-1-pentanol, 3-methyl-1-pentanol, 4-methyl-1-pentanol, 3-methyl-2-pentanol, 4-methyl-2-pentanol, 2-methyl-3-pentanol, 2,2-dimethyl-1-butanol, 2,3-dimethyl-1-butanol, 3,3-dimethyl-1-butanol, 3,3-dimethyl-2-butanol, 2-ethyl-1-butanol, and all structural isomers of heptanol, octanol and halogenated C₁-C₈-alkyl alcohols, particularly 2,2,2-trifluoroethanol. Particularly suitable are primary or secondary alcohols. Preferably primary alcohols are used as alcohols with one hydroxyl group. Particularly methanol, ethanol, 1-propanol, 2-propanol, 1-butanol, 2-butanol or 2,2,2-trifluoroethanol, preferably methanol, ethanol, 1-propanol, 1-butanol or 2,2,2-trifluoroethanol, are used as alcohols with one hydroxyl group.

In another embodiment the alcohol is a diol, hence has two hydroxyl groups. Preferably the diol is selected from the group consisting of ethane-1,2-diol, propane-1,2-diol, propane-1,3-diol, butane-1,4-diol, butane-1,3-diol, butane-1,2-diol, butane-2,3-diol, 2-methylpropane-1,2-diol, 2,2-dimethylpropane-1,3-diol, 1.2-dimethylpropane-1,3-diol, benzene-1,2-diol and cyclohexane-1,2-diols. From two cyclohexane-1,2-diols the preferred stereoisomer is *syn*-cyclohexane-1,2-diol (=*cis*-cyclohexane-1,2-diol).

The two hydroxyl group are in one embodiment bound to two adjacent carbon atoms, hence these diols are vicinal diols. Vicinal diols form a 5 membered ring in a ketal.

Particularly suitable are vicinal diols which are selected from the group consisting of ethane-1,2-diol, propane-1,2-diol, butane-1,2-diol, butane-2,3-diol, 2-methylpropane-1,2-diol, benzene-1,2-diol and *syn*-cyclohexane-1,2-diol, particularly ethane-1,2-diol.

Other particularly suitable are diols, in which the hydroxyl groups are separated by 3 carbon atoms, and, hence, form a very stable 6 membered ring in a ketal. Particularly suitable diols of this type are propane-1,3-diol, butane-1,3-diol, 2-methylpropane-1,3-diol, 2-methylbutane-1,3-diol, 2,2-dimethylpropane-1,3-diol, 1,2-dimethylpropane-1,3-diol, 3-methylpentane-2,4-diol and 2-(hydroxymethyl)-cyclohexanol.

Preferably primary alcohols are used as diols.

The reaction conditions and stoichiometry used for the ketal formation are known to the person skilled in the art.

The preferred ketals have the formula (XI) or (XII) 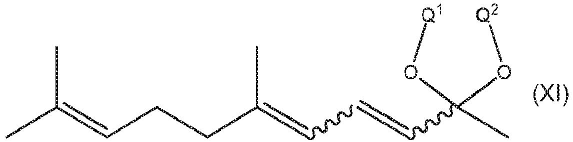 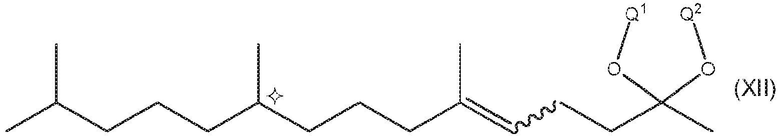 wherein a wavy line represents a carbon-carbon bond which is linked to the adjacent carbon-carbon double bond so as to have said carbon-carbon double bond either in the Z or in the E-configuration;
and wherein 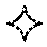 represents a stereogenic centre;
and wherein
Q¹ and Q²
stand either individually or both for a C₁-C₁₀ alkyl group or a halogenated C₁-C₁₀ alkyl group;
or form together a C₂-C₆ alkylene group or a C₆-C₈ cycloalkylene group.
Q¹ and Q² stand particularly for
either a linear C₁-C₁₀ alkyl group or fluorinated linear C₁-C₁₀ alkyl group, preferably a linear C₁-C₄ alkyl group or a -CH₂CF₃ group
or a group of formula 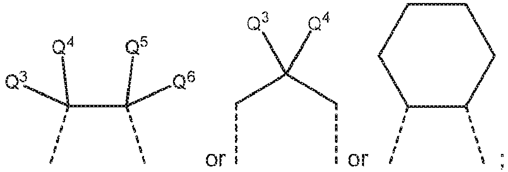 in which Q³, Q⁴, Q⁵ and Q⁶ are independently from each other hydrogen atoms or methyl or ethyl groups.

Particularly Q¹ and Q² stand both for a fluorinated linear C₁-C₁₀ alkyl group, preferably a linear C₁-C₄ alkyl group or a -CH₂CF₃ group or form together the alkylene group CH₂-C(CH₃)₂-CH₂.

Particularly suitable for ketals of formula (XI) are ketals selected of the group consisting of 10.10-dimethoxy-2,6-dimethylundeca-2,6,8-triene, 10,10-diethoxy-2,6-dimethylundeca-2,6,8-triene, 10,10- dipropoxy -2,6-dimethylundeca-2,6,8-triene, 10,10-diisopropoxy-2,6-dimethylundeca-2,6,8-triene, 10,10-di-sec-butoxy-2,6-dimethylundeca-2,6,8-triene, 10,10-bis(hexan-2-yloxy)-2,6-dimethylundeca-2,6,8-triene, 10.10- bis(isopentyloxy)-2.6-dimethylundeca-2.6,8-triene, 10,10- bis(pentyloxy)-2,6-dimethylundeca-2,6,8-triene, 10,10- bis(hexyloxy -2,6-dimethylundeca-2,6,8-triene, 2-(4,8-dimethylnona-1,3,7-trien-1-yl)-2-methyl-1,3-dioxolane, 2-(4,8-dimethylnona-1,3,7-trien-1-yl)-2,4-dimethyl-1,3-dioxolane, 2-(4,8-dimethylnona-1,3,7-trien-1-yl)-2,4,5-trimethyl-1,3-dioxolane, 2-(4,8-dimethylnona-1,3,7-trien-1-yl)-2-methylhexahydrobenzo[d][1,3]dioxole, 2-(4,8-dimethylnona-1,3,7-trien-1-yl)-2-methyl-1,3-dioxane, 2-(4,8-dimethylnona-1,3,7-trien-1-yl)-2,5-dimethyl-1,3-dioxane, 2-(4,8-dimethylnona-1,3,7-trien-1-yl)-2,5,5-trimethyl-1,3-dioxane, 2,6-dimethyl-10,10-bis(2,2,2-trifluoroethoxy)undeca-2,6,8-triene and all the EE-, EZ-, ZE- and ZZ-isomers thereof;

Preferably for the ketals of formula (XII) are ketals of the group consisting of 6,10,14-trimethyl-2,2-bis(2,2,2-trifluoroethoxy)pentadec-5-ene, 2,2-dimethoxy-6,10,14-trimethylpentadec-5-ene, 2,2-diethoxy-6,10,14-trimethylpentadec-5-ene, 2,2-dipropoxy-6,10,14-trimethylpentadec-5-ene, 2,2-dibutoxy-6,10,14-trimethylpentadec-5-ene, 2-methyl-2-(4,8.12-trimethyltridec-3-en-1-yl)-1,3-dioxolane, 2,4-dimethyl-2-(4,8,12-trimethyltridec-3-en-1-yl)-1,3-dioxolane, 2-methyl-2-(4,8,12-trimethyltridec-3-en-1-yl)-1,3-dioxane, 2,5-dimethyl-2-(4,8,12-trimethyltridec-3-en-1-yl)-1,3-dioxane, 2,5,5-trimethyl-2-(4,8,12-trimethyltridec-3-en-1-yl)-1,3-dioxane and all E- and Z- isomers thereof.

Particularly Q¹ and Q² stand both for a fluorinated linear C₁-C₁₀ alkyl group, preferably a linear C₁-C₄ alkyl group or a -CH₂CF₃ group or form together the alkylene group CH₂-C(CH₃)₂-CH₂.

Hence the preferred ketals to be asymmetrically hydrogenated are selected from the group consisting of 2,6-dimethyl-10,10-bis(2,2,2-trifluoroethoxy)undeca-2,6,8-triene, 2-(4,8-dimethylnona-1,3,7-trien-1-yl)-2,5,5-trimethyl-1,3-dioxane, 6,10,14-trimethyl-2,2-bis(2,2,2-trifluoroethoxy)pentadec-5-ene, 2,5,5-trimethyl-2-(4,8,12-trimethyltridec-3-en-1-yl)-1,3-dioxane all the EE-, EZ-, ZE- and ZZ-isomers or all E- and Z- isomers thereof thereof.

The hydrolysis of the hydrogenated ketal to the corresponding ketone is known to the person skilled in the art. Particularly suitable is the hydrolysis by means of an acid and isolation of the ketone formed, particularly by means of extraction.

### Asymmetric hydrogenation

Steps c) and f) involve asymmetric hydrogenations by molecular hydrogen in the presence of a chiral iridium complex.

Chiral iridium complexes are compounds having organic ligands being coordinated to a central iridium atom. The chirality of chiral iridium complexes is due to either the chirality of the ligands or the spacial arrangements of the ligands. This concept of chirality is well known from complex chemistry. Ligands can be monodentate or polydentate. Preferably, the ligands bound to the iridium central atom are chelating ligands. For the present invention, it has been shown that particularly chiral iridium complexes having an organic ligand bearing a stereogenic centre are very suitable.

It is preferred that the chiral iridium complex is bound to a chelating organic ligand having N and P as coordinating atoms and to either two olefins or to a diene having two carbon-carbon double bonds, and that, hence, the chiral iridium complex has preferably the following formula (III-0) 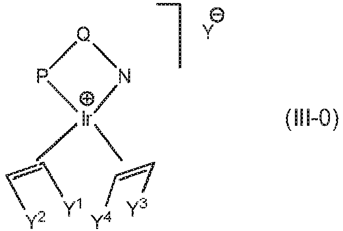 wherein
P-Q-N stands for a chelating organic ligand comprising a stereogenic centre or has planar or axial chirality and has a nitrogen and phosphorous atom as binding site to the iridium centre of the complex;
Y¹, Y², Y³ and Y⁴ are independently from each other hydrogen atoms, C₁₋₁₂-alkyl, C₅₋₁₀-cycloalkyl, or aromatic group; or at least two of them form together at least a two-valent bridged group of at least 2 carbon atoms; with the proviso that Y¹, Y², Y³ and Y⁴ are not all hydrogen atoms; and
Y^{Θ} is an anion, particularly selected from the group consisting of halide. PF₆⁻, SbF₆⁻, tetra(3,5-bis(trifluoromethyl)phenyl)borate (BAr_{F}⁻), BF₄⁻, perfluorinated sulfonates, preferably F₃C-SO₃⁻ or F₉C₄-SO₃⁻ ; ClO₄⁻, Al(OC₆F₅)₄⁻, Al(OC(CF₃)₃)₄⁻, N(SO₂CF₃)₂⁻N(SO₂C₄F₉)₂⁻ and B(C₆F₅)₄⁻.

The nitrogen and the phosphorous atom are preferably separated by 2 to 5. preferably 3, atoms in the chemical formula of the ligand P-Q-N.

The chelating organic ligand P-Q-N is preferably selected from the formulae (III-N1), (III-N2), (III-N3), (III-N4), (III-N5), (III-N6), (III-N7), (III-N8) and (III-N9) 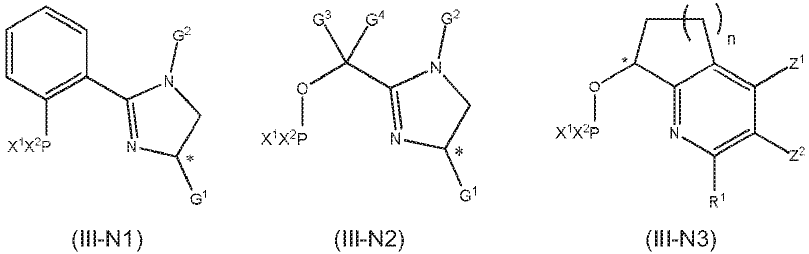 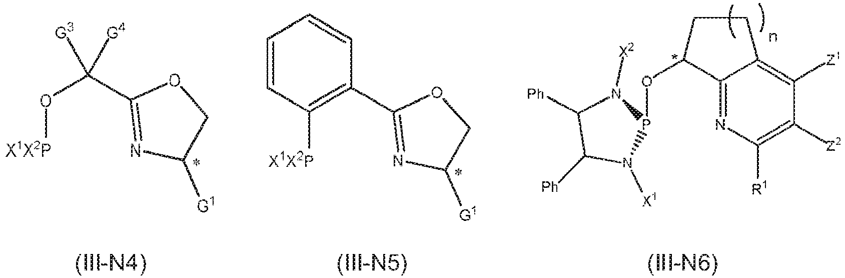 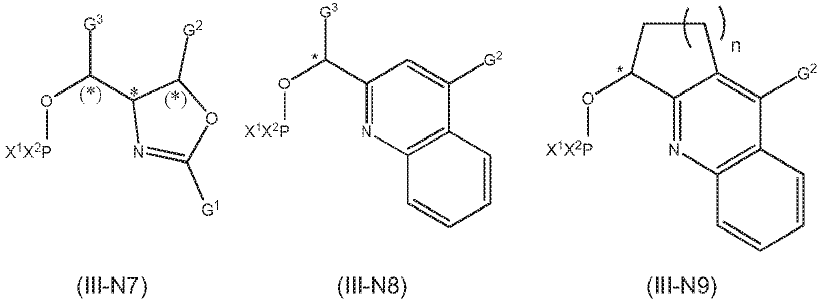 wherein G¹ represents either a C₁-C₄-alkyl, C₅₋₇-cycloalkyl, adamantyl, phenyl (optionally substituted with one to three C₁₋₅- alkyl, C₁₋₄-alkoxy, C₁₋₄-perfluoroalkyl groups and/or one to five halogen atoms)), benzyl, 1-naphthyl, 2-naphthyl, 2-furyl group;
G², G³ and G⁴ represent independently from each other hydrogen atoms or a C₁-C₄-alkyl, C₅₋₇-cycloalkyl, adamantyl, phenyl (optionally substituted with one to three C₁₋₅-, C₁₋₄-alkoxy, C₁₋₄-perfluoroalkyl groups and/or one to five halogen atoms)), benzyl, 1-naphthyl, 2-naphthyl, 2-furyl group;
X¹ and X² are independently from each other hydrogen atoms, C₁₋₄-alkyl, C₅₋₇-cycloalkyl, adamantyl, phenyl (optionally substituted with one to three C₁₋₅-alkyl, C₁₋₄-alkoxy, C₁₋₄-perfluoroalkyl groups and/or one to five halogen atoms)), benzyl, 1-naphthyl, 2-naphthyl, 2-furyl or ferrocenyl;
Ph stands for phenyl;
n is 1 or 2 or 3, preferred 1 or 2;
and R¹, Z¹ and Z² are as defined later for formula (III)

In case Y¹ and Y² and/or Y³ and Y⁴ form an olefin of the formula Y¹-=-Y² and/or of the Formula Y³-=-Y⁴, this olefin is or these olefins are preferably selected from the group consisting of ethene, prop-1-ene, 2-methylprop-1-ene, 2-methyl-but-2-ene, 2,3-dimethylbut-2-ene, (Z)-cyclooctene, cyclohexene, cycloheptene, cyclopentene and norbornene.

In case Y¹, Y², Y³ and Y⁴ are forming a diene, it is either cyclic (double bond in a cycle) or acyclic (double bond not in a cycle).

The two carbon-carbon double bonds of the diene are preferably linked by two carbon bonds, i.e. the dienes preferably comprise the substructure C=C-C-C-C=C.

Examples of preferred acylic dienes are hexa-1,5-diene, hepta-1,5-diene, octa-1,5-diene, octa-2,6-diene, 2,4-dialkyl-2,7-octadiene, 3,6-dialkylocta-2,6-diene, 1,2-divinylcyclohexane and 1,3-butadiene.

Examples for cyclic dienes are cycloocta-1,5-diene, cyclohexa-1,4-diene, cyclohexa-1,3-diene, 3,4,7,8-tetraalkylcycloocta-1,5-diene, 3,4,7-trialkylcycloocta-1,5-diene, 3,4-di-alkylcycloocta-1,5-diene, 3,7-di-alkylcycloocta-1,5-diene, 3,8-dialkylcycloocta-1,5-diene, 3-alkylcycloocta-1,5-diene; norbornadiene, 1-alkylnorbornadiene, 2-alkylnorbornadiene, 7-alkylnorbornadiene, dicyclopentadiene, cyclopentadiene and (1s,4s)-bicyclo[2.2.2]octa-2,5-diene.

Preferred diene is cycloocta-1,5-diene.

A highly preferred class of chiral iridium complexes are chiral iridium complexes of formula (III) 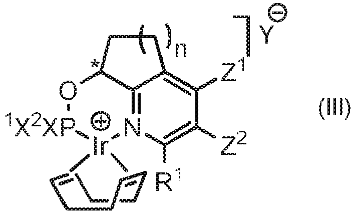 wherein
n is 1 or 2 or 3. preferred 1 or 2;
X¹ and X² are independently from each other hydrogen atoms, C₁₋₄-alkyl, C₅₋₇-cycloalkyl, adamantyl, phenyl (optionally substituted with one to three C₁₋₅-, C₁₋₄-alkoxy, C₁₋₄-perfluoroalkyl groups and/or one to five halogen atoms)), benzyl, 1-naphthyl, 2-naphthyl, 2-furyl or ferrocenyl;
Z¹ and Z² are independently from each other hydrogen atoms, C₁₋₅-alkyl or C₁₋₅-alkoxy groups;
or Z¹ and Z² stand together for a bridging group forming a 5 to 6 membered ring;
Y^{Θ} is an anion, particularly selected from the group consisting of halide, PF₆⁻, SbF₆⁻, tetra(3,5-bis(trifluoromethyl)phenyl)borate (BAr_{F}⁻), BF₄⁻, perfluorinated sulfonates, preferably F₃C-SO₃⁻ or F₉C₄-SO₃⁻ ; ClO₄⁻, Al(OC₆F₅)₄⁻, Al(OC(CF₃)₃)₄⁻, N(SO₂CF₃)₂⁻N(SO₂C₄F₉)₂⁻ and B(C₆F₅)₄⁻;
R¹ represents either phenyl or o-tolyl or m-tolyl or p-tolyl or a group of formula (IVa) or (IVb) or (IVc) 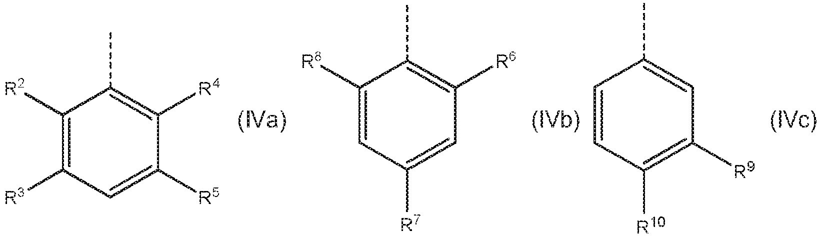
   wherein R² and R³ represent either both H or a C₁-C₄-alkyl group or a halogenated C₁-C₄-alkyl group or represent a divalent group forming together a 6-membered cycloaliphatic or an aromatic ring which optionally is substituted by halogen atoms or by C₁-C₄-alkyl groups or by C₁-C₄-alkoxy groups
   R⁴ and R⁵ represent either both H or a C₁-C₄-alkyl group or a halogenated C₁-C₄-alkyl group or a divalent group forming together a 6-membered cycloaliphatic or an aromatic ring which optionally is substituted by halogen atoms or by C₁-C₄-alkyl groups or by C₁-C₄-alkoxy groups;
   R⁶ and R⁷ and R⁸ represent each a C₁-C₄-alkyl group or a halogenated C₁-C₄-alkyl group;
   R⁹ and R¹⁰ represent either both H or a C₁-C₄-alkyl group or a halogenated C₁-C₄-alkyl group or a divalent group forming together a 6-membered cycloaliphatic or an aromatic ring which optionally is substituted by halogen atoms or by C₁-C₄-alkyl groups or by C₁-C₄-alkoxy groups;
   and wherein * represents a stereogenic centre of the complex of formula (III).

The complex of formula (III) is neutral, i.e. the complex consists of a complex cation of formula (III') and anion Y as defined before.

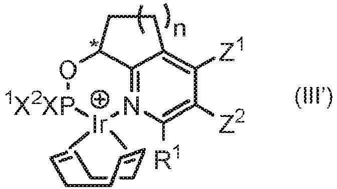

The person skilled in the art knows that anions and cations may be dissociated.

X¹ and/or X² represent preferably hydrogen atoms, methyl, ethyl, n-propyl, iso-propyl, n-butyl, iso-butyl, tert-butyl, n-pentyl, iso-pentyl, neopentyl, cyclopentyl, cyclohexyl, cycloheptyl, adamantly, phenyl, benzyl, o-tolyl, m-tolyl, p-tolyl, 4-methoxyphenyl, 4-trifluoromethylphenyl, 3,5-di-tert-butylphenyl, 3,5-dimethoxyphenyl, 1-naphthyl, naphthyl, 2-furyl, ferrocenyl or a phenyl group which is substituted with one to five halogen atoms.

In case of X¹ and/or X² representing phenyl groups which are substituted with one to five halogen atoms, the phenyl groups substituted by fluorine atoms are particularly useful, i.e. C₆H₄F, C₆H₃F₂, C₆H₂F₃, C₆HF₄ or C₆F₅.

In case of X¹ and/or X² representing phenyl groups which are substituted with one to three C₁₋₄-alkyl, the phenyl groups substituted by methyl group(s) are particularly useful, particularly ortho-tolyl and para-tolyl.

Preferably both X¹ and X² represent the same substituent.

Most preferred both X¹ and X² are phenyl or ortho-tolyl groups.

It is preferred that the C₁-C₄-alkyl or alkoxy groups used in the definition of R², R³, R⁴, R⁵ R⁶, R⁷, R⁸, R⁹ and R¹⁰ above are primary or secondary, preferably primary, alkyl or alkoxy groups.

A particularly suited substituent R¹ of formula (IVa) is the 9-anthryl or 1-naphthyl group.

A further particularly suited substituent R¹ of formula (IVb) is the mesityl group.

A further particularly suited substituent R¹ of formula (IVc) is the 2-naphthyl group.

Preferably R¹ is represented by phenyl (abbreviated as "Ph") or formula (IV-1) or (IV-2) or (IV-3), particularly (IV-1) or (IV-3).

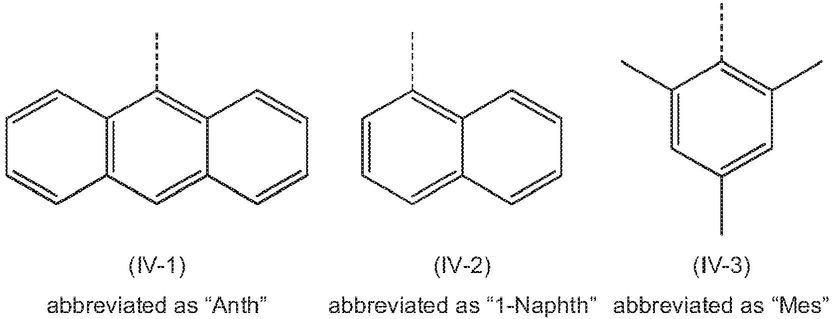

It has been found that the most preferred substituent R¹ is either 9-anthryl or phenyl.

The preferred chiral iridium complexes of formula (III) are the complexes of formulae (III-A), (III-B), (III-C), (III-D), (III-E) and (III-F).

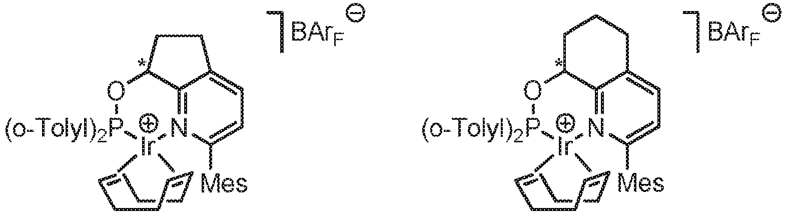

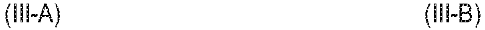

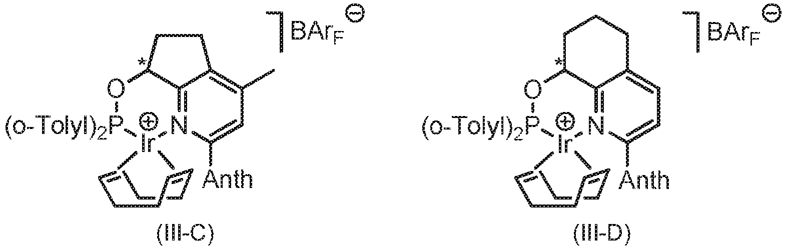

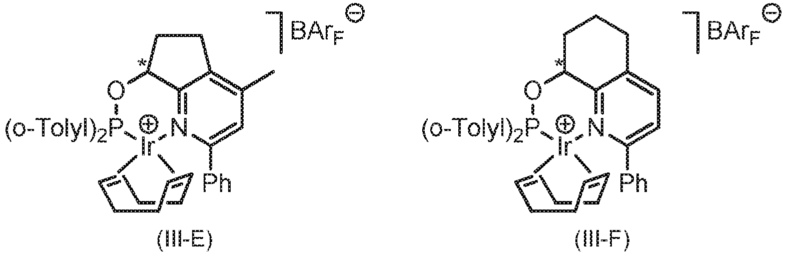

Most preferred as chiral iridium complexes of formula (III) are the complexes of formulae (III-C) and (III-D) and (III-F), particularly the one of formula (III-C) or (III-F).

The chiral iridium complexes of formula (III) can be synthesized accordingly as described in detail in Chem. Sci., 2010, 1, 72 - 78.

The iridium complex of formula (III) is chiral. The chirality at said chiral centre marked by the asterisk is either S or R, i.e. there exist two enantiomers (IIIa) and (IIIb) of the chiral complex of formula (III):

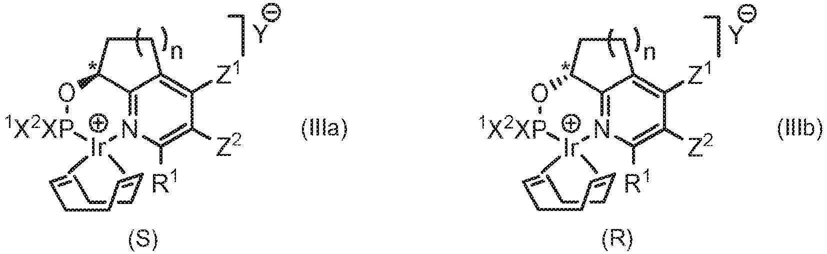

The individual enantiomers of the complex of formula (III) could be principally separated after the complexation step from a racemic mixture. However, as Chem. Sci., 2010, 1, 72 - 78 discloses, the synthesis of the complex of formula (III) comprises a reaction involving a non-racemic chiral alcohol. As it is known that the further reaction steps do not modify the chirality of the complex its isomeric purity (S:R-ratio) is governed therefore by the enantiomeric purity of said alcohol. As said corresponding alcohol can be obtained in a R/S ratio of more than 99 % resp. lower than 1 %, the complex of formula (III) can be obtained in extremely high enantiomeric purities, particularly in a R/S ratio of more than 99 % resp. lower than 1 %.

The chiral iridium complex is preferably used in an excess of one enantiomer.

Particularly, it is preferred that the ratio of the molar amounts of the individual enantiomers R:S of the chiral iridium complex of formula (III) is more than 90 : 10 or less than 10 : 90, preferably in the range of 100 : 0 to 98 : 2 or 0 : 100 to 2 : 98. Most preferred is that this ratio is about 100 : 0 resp. about 0 : 100. The ultimately preferred ratio is 100 : 0 resp. 0 : 100.

In one embodiment the stereogenic centre indicated by * has the R-configuration.

In another embodiment the stereogenic centre indicated by * has the S-configuration.

The hydrogenating agent is molecular hydrogen (H₂).

The amount of chiral iridium complex is preferably from about 0.0001 to about 5 mol %, preferably from about 0.001 to about 2 mol %, more preferably from about 0.01 to about 1 mol %, based on the amount of the ketone resp. ketal.

The hydrogenation can be carried out in substance or in an inert carrier, particularly in an inert solvent, or a mixture of inert solvents. The hydrogenation is preferred carried out in substance (neat).

Preferred suitable solvents are halogenated hydrocarbons, hydrocarbons, carbonates, ethers and halogenated alcohols.

Particularly preferred solvents are hydrocarbons, fluorinated alcohols and halogenated hydrocarbons, particularly halogenated aliphatic hydrocarbons.

Preferred examples of hydrocarbons are hexane, heptane, toluene, xylene and benzene, particularly toluene and heptane.

Preferred ethers are dialkylethers. Particularly useful ethers are dialklyethers with less than 8 carbon atoms. Most preferred ether is methyl *tert*.-butyl ether (CH₃-O-C(CH₃)₃).

Preferred halogenated alcohols are fluorinated alcohols. A particularly preferred fluorinated alcohol is 2,2,2-trifluoroethanol.

One preferred group of halogenated hydrocarbon are halogenated aromatic compounds, particularly chlorobenzene.

Preferred examples of halogenated aliphatic hydrocarbons are mono- or polyhalogenated linear or branched or cyclic C₁- to C₁₅-alkanes. Especially preferred examples are mono- or polychlorinated or -brominated linear or branched or cyclic C₁- to C₁₅-alkanes. More preferred are mono- or polychlorinated linear or branched or cyclic C₁- to C₁₅ -alkanes. Most preferred are dichloromethane, 1,2-dichloroethane, 1,1,1-trichloroethane, chloroform, and methylene bromide.

The most preferred solvent for the hydrogenation is dichloromethane.

The amount of solvent used is not very critical. However, it has been shown that the concentration of the ketone or ketal to be hydrogenated is preferably between 0.05 and 1 M, particularly between 0.2 and 0.7 M.

The hydrogenation reaction is conveniently carried out at an absolute pressure of molecular hydrogen from about 1 to about 100 bar, preferably at an absolute pressure of molecular hydrogen from about 20 to about 75 bar. The reaction temperature is conveniently between about 0 to about 100°C, preferably between about 10 to about 60°C.

The sequence of addition of the reactants and solvent is not critical.

The technique and apparatus suitable for the hydrogenation is principally known to the person skilled in the art.

By the asymmetric hydrogenation a prochiral carbon-carbon double bond is hydrogenated to form a chiral stereogenic centre at one or both of the carbon atoms.

In step c) either 6,10-dimethylundeca-3,5,9-trien-2-one or a ketal of 6,10-dimethylundeca-3,5,9-trien-2-one is asymmetrically hydrogenated.

In step f) either 6,10,14-trimethylpentadec-5-en-2-one or a ketal of 6,10,14-trimethylpentadec-5-en-2-one is hydrogenated.

In case a ketal is asymmetrically hydrogenated, after the asymmetric hydrogenation the asymmetrically hydrogenated ketal has preferably the formula (XV) or (XVI). 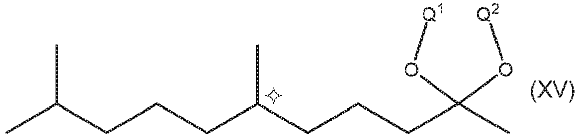 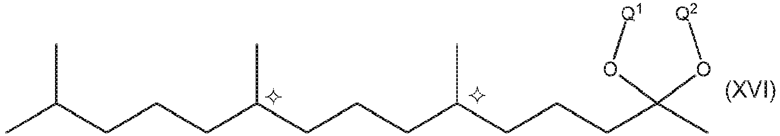 wherein 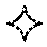 represents a stereogenic centre;
and wherein Q¹ and Q² are as defined for formula (XI) and (XII).

Hence the preferred ketals which have been asymmetrically hydrogenated are preferably selected from the group consisting of 2-(4,8-dimethylnonyl)-2,5,5-trimethyl-1,3-dioxane, 6,10-dimethyl-2,2-bis(2,2,2-trifluoroethoxy)undecane, 2,5,5-trimethyl-2-(4,8,12-trimethyltridecyl)-1,3-dioxane, 6,10,14-trimethyl-2,2-bis(2,2,2-trifluoroethoxy)pentadecane;

(R)-2-(4,8-dimethylnonyl)-2,5,5-trimethyl-1,3-dioxane, (R)-6,10-dimethyl-2,2-bis(2,2,2-trifluoroethoxy)undecane, 2,5,5-trimethyl-2-((4R,8R)-4,8,12-trimethyltridecyl)-1,3-dioxane and (6R,10R)-6,10,14-trimethyl-2,2-bis(2,2,2-trifluoroethoxy)pentadecane.

When these ketals are hydrolysed into the corresponding ketone, they yield 6,10-dimethylundecan-2-one or 6,10,14-trimethylpentadecan-2-one, or (R)-6,10-dimethylundecan-2-one or (6R,10R)-6,10,14-trimethylpentadecan-2-one, respectively.

Despite the fact that the asymmetric hydrogenation of 6,10-dimethylundeca-3,5,9-trien-2-one and (R)-6,10,14-trimethylpentadec-5-en-2-one by means of molecular hydrogen in the presence of a chiral iridium complex, particularly those of formula (III), is already rather fast and efficient and shows high conversion rates as well as excellent selectivities, it has been observed that the asymmetric hydrogenation can even be improved when ketals of the corresponding ketones are asymmetrically hydrogenated.

It has been observed that the chiral iridium complex of a specific chirality (R or S) converts the starting material into a product bearing a specific stereogenic centre, which is formed as a result of the asymmetric hydrogenation.

As is in the present invention it is desired to produce products bearing a stereocentre with R-configuration by asymmetric hydrogenation i.e. (R)-6,10-dimethylundecan-2-one in step c), and (6R,10R)-6,10,14-trimethylpentadecan-2-one in step f), respectively, the chirality of the chiral iridium complex needs to be selected depending on whether the olefin isomers being separated in step b) and step e), respectively, have the Z- or E-configuration.

It has been shown that when chiral iridium complexes of formula (III) having the S-configuration at the stereogenic centre indicated by * are used for the hydrogenation of E-isomers, i.e. (3E,5E)- and (3Z,5E)-6,10-dimethylundeca-3,5,9-trien-2-one and (R,E)-6,10.14-trimethylpentadec-5-en-2-one, respectively, the corresponding products, i.e. (R)-6,10-dimethylundecan-2-one in step c), and (6R,10R)-6,10,14-trimethylpentadecan-2-one in step f), are obtained bearing the R-configuration at the newly formed stereogenic centre. Correspondingly, the hydrogenation of Z-isomers, i.e. (3E,ZE)- and (3Z,5Z)-6,10-dimethylundeca-3,5,9-trien-2-one and (R,Z)-6,10,14-trimethylpentadec-5-en-2-one, respectively, in the presence of the chiral iridium complex of formula (III) having the R-configuration at the stereogenic centre indicated by * furnishes the same products, i.e. (R)-6,10-dimethylundecan-2-one in step c), and (6R,10R)-6,10,14-trimethylpentadecan-2-one in step f), are obtained bearing the R-configuration at the newly formed stereogenic centre

Surprisingly, it has been found that this finding is independent from whether a ketone or a ketal is used in step c) or f).

Therefore, the chiral iridium complex of formula (III) used in step c) and/or f) for the asymmetric hydrogenation preferably has the
S-configuration at the stereogenic centre indicated by * in case (3E,5E)-or (3Z,5E)-6,10-dimethylundeca-3,5,9-trien-2-one or ketals thereof, or (R,E)-6,10,14-trimethypentadec-5-en-2-one, or a ketal thereof, are to be hydrogenated;
or has the
R-configuration at the stereogenic centre indicated by * in case (3E,5Z)-or (3Z,5Z)-6,10-dimethylundeca-3,5,9-trien-2-one or ketals thereof, or (R,Z)-6,10,14-trimethylpentadec-5-en-2-one, or a ketal thereof, are to be hydrogenated.

In a preferred embodiment of the invention the asymmetric hydrogenation in step c) and/or step f) takes place in the presence of an additive which is selected from the group consisting of organic sulfonic acids, transition metal salts of organic sulfonic acids, metal alkoxides, aluminoxanes, alkyl aluminoxanes and B(R)₍₃₋ᵥ₎(OZ)ᵥ; wherein v stands for 0, 1, 2 or 3 and R stands for F, a C₁₋₆-alkyl, a halogenated C₁₋₆-alkyl, an aryl or halogenated aryl group; and Z stands a C₁₋₆-alkyl, a halogenated C₁₋₆-alkyl, an aryl or halogenated aryl group.

Particularly suitable additives are selected from the group consisting of triflic acid, alkyl aluminoxanes, particularly methyl aluminoxane, ethyl aluminoxane, tetra alkoxy titanates, B(R)₍₃₋ᵥ₎(OZ)ᵥ; particularly tri-*iso*propylborate and triethylborane and BF₃, preferably in the form of a BF₃ etherate.

Particularly useful as the transition metal salts of organic sulfonic acids are scandium, indium, yttrium and zirconium salts of organic sulfonic acids.

Metal alkoxides are known to the person skilled in the art. This term particularly relates to the alkoxides of the elements of the group 4 and 13 of the periodic system. It is also known to the person skilled in the art that the metal alkoxides often do not form well-defined structures. Characteristically, metal alkoxides have hydrocarbyl group bound by an oxygen atom to a metal centre. A metal alkoxide may also have different metal centres which are bridged by oxygen or oxygen containing groups, such as for example (polynuclear) aluminium oxoalkoxides.

Particularly useful as metal alkoxides are titanium alkoxides (also being called alkoxy titanates) zirconium alkoxides (also being called alkoxy zirconates) or aluminium alkoxides.

A particularly preferred class of metal alkoxide is of the type of polynuclear aluminium oxoalkoxides such as disclosed in J. Chem. Soc.. Dalton Trans., 2002, 259-266 or in Organometallics 1993, 12, 2429-2431.

Alkyl aluminoxanes, are known products which are particularly useful as co-catalysts for olefin polymerizations of the Ziegler-Natta type. They are prepared by controlled hydrolysis of trialkylaluminium compound, particularly trimethylaluminium or triethylaluminium. The hydrolysis can be achieved for example by hydrated metal salts (metal salts containing crystal water).

Preferably the additive is selected from the group consisting of triflic acid, alkyl aluminoxanes, particularly methyl aluminoxane, ethyl aluminoxane, tetra alkoxy titanates, B(R)₍₃₋ᵥ₎(OZ)ᵥ; particularly tri-*iso*propylborate and triethylborane and BF₃, preferably in the form of a BF₃ etherate.

More preferred are triflic acid, alkyl aluminoxanes, particularly methyl aluminoxane, ethyl aluminoxane, tetra alkoxy titanates. B(R)₍₃₋ᵥ₎(OZ)ᵥ; particularly tri-*iso*propylborate and triethylborane.

Especially good results have been obtained by an additive with has been obtained from trimethylaluminoxane and 2,2,2-trifluoroethanol or from trialkylaluminium and 2,2,2-trifluoroethanol.

It has been found that the quality and speed of the asymmetric hydrogenation using molecular hydrogen in the presence of a chiral iridium complex is enhanced significantly when the above mentioned additives are used.

It has been further observed that, most significantly, the efficiency of the asymmetric hydrogenation is maximized when the above mentioned additives are used with the corresponding ketal of the ketones to be asymmetrically hydrogenated. i.e. ketals of 6,10-dimethylundeca-3,5,9-trien-2-one and (R)-6,10,14-trimethylpentadec-5-en-2-one.

The increased efficiency has the effect that the amount of chiral iridium complex can be remarkably lowered by using an ketal of 6,10-dimethylundeca-3,5,9-trien-2-one or (R)-6,10,14-trimethylpentadec-5-en-2-one. and/or addition of the mentioned additive(s), particularly in the combination with fluorinated alcohols, particularly 2,2,2-trfluoroethanol, to achieve a given yield and stereospecific hydrogenation in the asymmetric hydrogenation as compared to the corresponding asymmetric hydrogenation of 6,10-dimethylundeca-3,5,9-trien-2-one and (R)-6,10,14-trimethylpentadec-5-en-2-one as such.

When the process comprises steps of cis/trans isomerization, as discussed above in detail, the process of invention is extremely interesting because for an optimal use of all starting material, it is not necessary to set up two parallel product lines for the separate asymmetric hydrogenation of each isomer using hydrogenation complexes of opposite chirality. Therefore, the *in-situ isomerization*, as discussed above, is much preferred.

### Chemical transformation

In step d) (R)-6,10-dimethylundecan-2-one is chemically transformed to a mixture of (R,E)-6,10,14-trimethylpentadec-5-en-2-one and (R,Z)-6,10,14-trimethylpentadec-5-en-2-one ;

This chemical transformation can be done in different ways.

In a preferred method the chemical transformation of (R)-6,10-dimethylundecan-2-one to a mixture of (R,E)-6,10,14-trimethylpentadec-5-en-2-one and (R,Z)-6,10,14-trimethylpentadec-5-en-2-one in step d) is done by the steps
either
d1) ethynylation of (R)-6,10-dimethylundecan-2-one using ethyne in the presence of a basic substance to yield (7R)-3,7,11-trimethyldodec-1-yn-3-ol;
d2) hydrogenation of (7R)-3,7,11-trimethyldodec-1-yn-3-ol with molecular hydrogen in the presence of a Lindlar catalyst to yield (7R)-3,7,11-trimethyldodec-1-en-3-ol;
or
d1')vinylation of (R)-6,10-dimethylundecan-2-one by addition of a vinyl Grignard reagent to yield (7R)-3,7,11-trimethyldodec-1-en-3-ol;
followed by
either
d3) reacting (7R)-3,7,11-trimethyldodec-1-en-3-ol with 2-methoxyprop-1-ene to yield a mixture of (R,E)-6,10,14-trimethylpentadec-5-en-2-one and (R,Z)-6,10,14-trimethylpentadec-5-en-2-one;
or
d3')reacting (7R)-3,7,11-trimethyldodec-1-en-3-ol with an alkyl acetoacetate or diketene in the presence of a base and/or an acid to yield a mixture of (R,E)-6,10,14-trimethylpentadec-5-en-2-one and (R,Z)-6,10,14-trimethylpentadec-5-en-2-one.

Details for the reaction type and conditions of the variant using steps d1) is disclosed in EP 1 532 092 B1, particularly in example 2, or WO 2003/029175 A1 (using a basic anion exchange resin).

The hydrogenation with molecular hydrogen in the presence of a Lindlar catalyst in step d2) is known to the person skilled in the art. For example A. Ofner et al, Helv. Chim. Acta 1959, 42, 2577-2584 disclose the combination of steps d1) and d2).

US 4,028,385 for example discloses details for the reaction type and conditions of the variant using step d1') as well as also for steps d1) and d2).

Details for the reaction type and conditions of the variant using step d3), which is a Saucy-Marbet reaction, are disclosed for example in DE 1193490 and DE 196 49 564 A1.

Details for the reaction type and conditions of the first variant in step d3'), which is a Carroll rearrangement, are disclosed for example in chapter 8 "The Carroll Rearrangement" in Hatcher et al., The Claisen Rearrangement; Hiersemann, M.; Nubbemeyer, U., Eds.; Wiley-VCH Verlag GmbH & Co. KGaA, 2007; 397-430.

In the Carroll rearrangement the reaction occurs with an alkyl acetoacetate, preferably methyl acetoacetate or ethyl acetoacetate, in the presence of a base, preferably an alkali acetate such as sodium acetate.

The reaction type and conditions of the second variant in step d3') are disclosed for example in US 2,795,617 or in W. Kimel et al., J. Org. Chem. 1957, 22, 1611-1618.

The reaction preferably occurs with diketene in the presence of preferably pyridine and acetic acid resp. in the presence of an alkoxide.

As mentioned earlier, (6R, 10R)-6,10,14-trimethylpentadecan-2-one is an important intermediate and is particularly useful for the synthesis of (R,R)-isophytol, (2-*ambo*)-α-tocopherol or of (2R,4'R,8'R)-(α-tocopherol.

Therefore, in a further aspect the invention relates to a process of manufacturing (R,R)-isophytol ((3RS,7R,11R)-3,7,11,15-tetramethylhexadec-1-en-3-ol) which comprises the process of manufacturing (6R,10R)-6,10,14-trimethylpentadecan-2-one as described above in detail: followed by the steps either
g) ethynylation of (6R,10R)-6,10,14-trimethylpentadecan-2-one using ethyne in the presence of a base to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R, R)-isophytol;
   or
h') vinylation of (6R,10R)-6,10,14-trimethylpentadecan-2-one by addition of a vinyl Grignard reagent to yield (R,R)-isophytol.

The conditions for steps g) and h) and h') correspond to the ones described for the analogous steps d1) and d2) and d1') in step d).

In a further aspect the invention relates to a process of manufacturing compound of formula (V) comprising
the process of manufacturing (6R,10R)-6,10,14-trimethylpentadecan-2-one as described above in detail;
followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
   or
h') vinylation of (6R,10R)-6,10,14-trimethylpentadecan-2-one by addition of a vinyl Grignard reagent to yield (R,R)-isophytol;
followed by the steps
m) condensing (R,R)-isophytol with compound of formula (VI) to yield compound of formula (V) being an isomeric mixture in view of the chirality at the centre indicated by #; 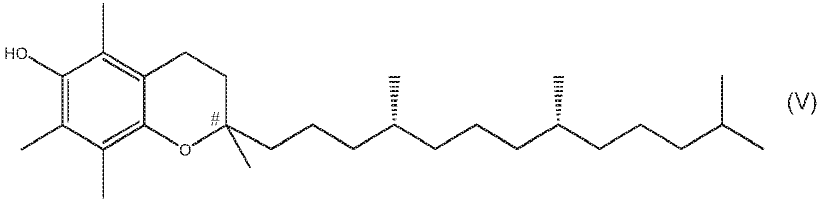 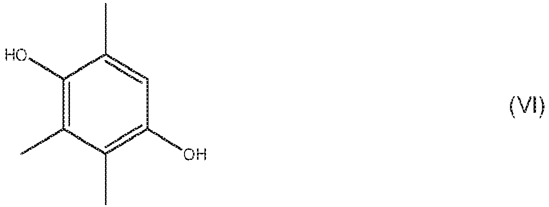 wherein # represents a stereogenic centre.

The conditions for steps g) and h) and h') correspond to the ones described for the analogous steps d1) and d2) and d1') in step d).

The condensation reaction of (R,R)-isophytol and compound of formula (VI), described as step m), is known by the person skilled in the art. For this condensation a series of catalysts may be used such as ZnCl₂/mineral acid, BF₃/AlCl₃, Fe/HCl, trifluoroacetic acid or boric acid/carboxylic acid as well as indium(III) or scandium(III) salts as disclosed in WO 2005/121115 A1. Furthermore, suitable catalysts are heteropoly acids, particularly 12-tungstophosphoric acid or 12-tungstosilicic acid such as disclosed in EP 0 970 953 A1.

The compounds of formula (V) represent (2-*ambo*)-(α-tocopherol, i.e. a mixture of the corresponding (2R,4'R,8'R)-α-tocopherol and (2S,4'R,8'R)-α-tocopherol).

In a further aspect the invention relates to a process of manufacturing compound of formula (V-A) comprising
the process of manufacturing (6R,10R)-6,10,14-trimethylpentadecan-2-one as described above in detail;
followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R, R)-isophytol;
   or
h') vinylation of (6R,10R)-6,10,14-trimethylpentadecan-2-one by addition of a vinyl Grignard reagent to yield (R,R)-isophytol;
followed by the steps
m) condensing (R,R)-isophytol with compound of formula (VI) to yield compound of formula (V) being an isomeric mixture in view of the chirality at the centre indicated by #; 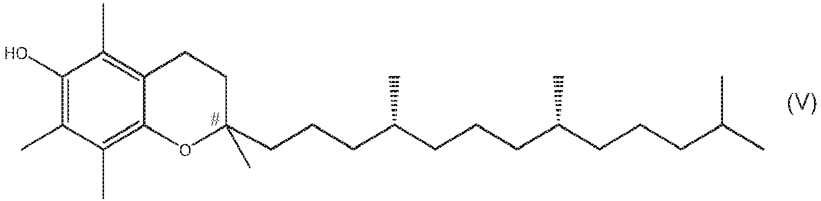 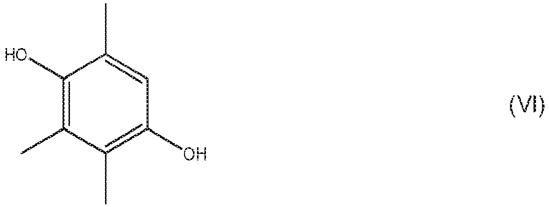
wherein # represents a stereogenic centre; and
n) isolating compound of formula (V-A) from the isomeric mixture of formula (V)

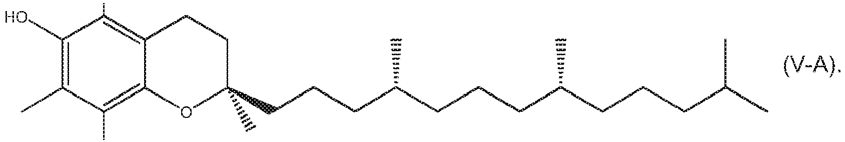

This process of manufacturing compound of formula (V-A) is the same as the process of manufacturing compound of formula (V) except for an additional step n).

The isolation of a (2R,4'R,8'R)-α-tocopherol from the corresponding (2-*ambo*)-α-tocopherol can be achieved by chromatographic separation by means of a chiral phase, particularly as described in WO2012/152779 A1. It is also preferred to enhance the yield in (2R,4'R,8'R)-α-tocopherol by means of epimerization of fractions enriched in (2S,4'R,8'R)-α-tocopherol as disclosed as step c) in WO2O1211152779 A1.

The substances ((3E,5E)-/ (3E,5Z)-/ (3Z,5E)-/ and (3Z,5Z)-6,10-dimethylundeca-3,5,9-trien-2-one; ketals of (3E,5E)-/ (3E,5Z)-/ (3Z,5E)-/ and (3Z,5Z)-6,10-dimethylundeca-3,5,9-trien-2-one, (R,E)-6,10,14-trimethylpentadec-5-en-2-one, (R,Z)-6,10,14-trimethylpentadec-5-en-2-one, ketals of (R,E)-6,10,14-trimethylpentadec-5-en-2-one, ketals of (R,Z)-6,10,14-trimethylpentadec-5-en-2-one, (6R,10R)-6,10,14-trimethylpentadecan-2-one, ketals of (6R,10R)-6,10,14-trimethylpentadecan-2-one, (7R)-3,7,11-trimethyldodec-1-yn-3-ol, (7R)-3,7,11-trimethyldodec-1-en-3-ol and (R,R)-isophytol are important intermediates for the synthesis of tocopherols, vitamin K1. as well as for flavours and fragrances or for pharmaceutical products. The majority of them has a typical odour which makes them very attractive to be used as ingredients in products of the industry of flavours and fragrances such as in perfumes.

In a further aspect, the invention relates to a composition as defined in claim 13 comprising
- at least one ketal of formula (XI) and
- at least one chiral iridium complex.

The ketal of formula (XI) and the chiral iridium complex, their ratios and as well their preferred embodiments, properties and effects have been discussed in this documents already in great detail.

In a final aspect, the present disclosure relates to ketals of of formula (XX-A) or (XX-B) or (XX-C) or (XX-D) or (XX-E) 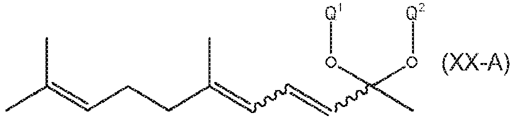 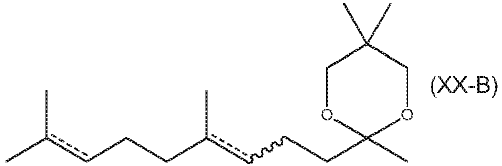 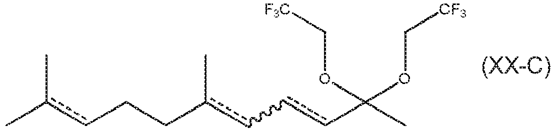 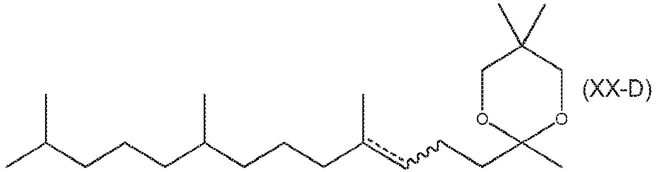 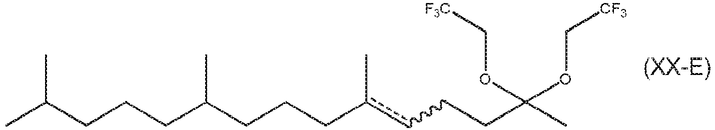 wherein the double bond having dotted lines (------) in the above formulae represents either a single carbon-carbon bond or a double carbon-carbon bond;and
wherein a wavy line represents a carbon-carbon bond which is linked to an adjacent single carbon bond (------ representing -) or to an adjacent carbon-carbon double bond (------ representing 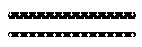 ) so as to have said carbon-carbon double bond either in the Z or in the E-configuration;
and wherein
Q¹ and Q²
stand either individually or both for a C₁-C₁₀ alkyl group or a halogenated C₁-C₁₀ alkyl group;
or form together a C₂-C₆ alkylene group or a C₆-C₈ cycloalkylene group;
with the exception of 2-(4,8-dimethylnona-1,3,7-trien-1-yl)-2-methyl-1,3-dioxolane.
Particularly the acetal or ketal is selected from the group consisting of 10,10-dimethoxy-2,6-dimethylundeca-2,6,8-triene, 10,10-diethoxy-2.6-dimethylundeca-2,6,8-triene, 10,10- dipropoxy -2,6-dimethylundeca-2,6,8-triene, 10,10- diisopropoxy -2,6-dimethylundeca-2,6,8-triene, 10,10-di-sec-butoxy -2,6-dimethylundeca-2,6,8-triene, 10,10-bis(hexan-2-yloxy)-2,6-dimethylundeca-2,6,8-triene, 10,10- bis(isopentyloxy)-2,6-dimethylundeca-2.6,8-triene. 10,10- bis(pentyloxy)-2,6-dimethylundeca-2,6.8-triene, 10,10-bis(hexyloxy -2,6-dimethylundeca-2,6,8-triene, 2-(4,8-dimethylnona-1,3,7-trien-1-yl)-2,4-dimethyl-1,3-dioxolane, 2-(4,8-dimethylnona-1,3,7-trien-1-yl)-2,4,5-trimethyl-1,3-dioxolane, 2-(4,8-dimethylnona-1,3,7-trien-1-yl)-2-methylhexahydrobenzo[d][1,3]dioxole, 2-(4,8-dimethylnona-1,3,7-trien-1-yl)-2-methyl-1,3-dioxane, 2-(4,8-dimethylnona-1,3,7-trien-1-yl)-2,5-dimethyl-1,3-dioxane, 2-(4,8-dimethylnona-1,3,7-trien-1-yl)-2,5,5-trimethyl-1,3-dioxane, 2,6-dimethyl-10,10-bis(2,2,2-trifluoroethoxy)undeca-2,6,8-triene and all the EE-, EZ-, ZE- and ZZ-isomers thereof;
or selected from the group consisting of 6,10,14-trimethyl-2,2-bis(2,2,2-trifluoroethoxy)pentadec-5-ene, 2,5,5-trimethyl-2-(4,8,12-trimethyltridec-3-en-1-yl)-1,3-dioxane and all E- and Z- isomers thereof.

Most preferred are the ketals of formulae (XX-C) and (XX-E).

Hence the preferred ketals to be asymmetrically hydrogenated are selected from the group consisting of 2,6-dimethyl-10,10-bis(2,2,2-trifluoroethoxy)undeca-2,6,8-triene, 2-(4,8-dimethylnona-1,3,7-trien-1-yl)-2,5,5-trimethyl-1,3-dioxane, 6,10,14-trimethyl-2,2-bis(2,2,2-trifluoroethoxy)pentadec-5-ene, 2,5,5-trimethyl-2-(4,8,12-trimethyltridec-3-en-1-yl)-1,3-dioxane and all the EE-, EZ-, ZE- and ZZ-isomers or all E- and Z- isomers thereof thereof.

All these ketals are particularly suited for the asymmetric hydrogenation as described above in detail or are the product of said asymmetric hydrogenation. As mentioned also before the ketals of unsaturated ketones behave extremely advantageously as compared to the corresponding ketones.

In as far as ketals as such are concerned, the part of the present disclosure which forms the invention for which protection is sought refers to ketals as defined in claim 14.

### Figures

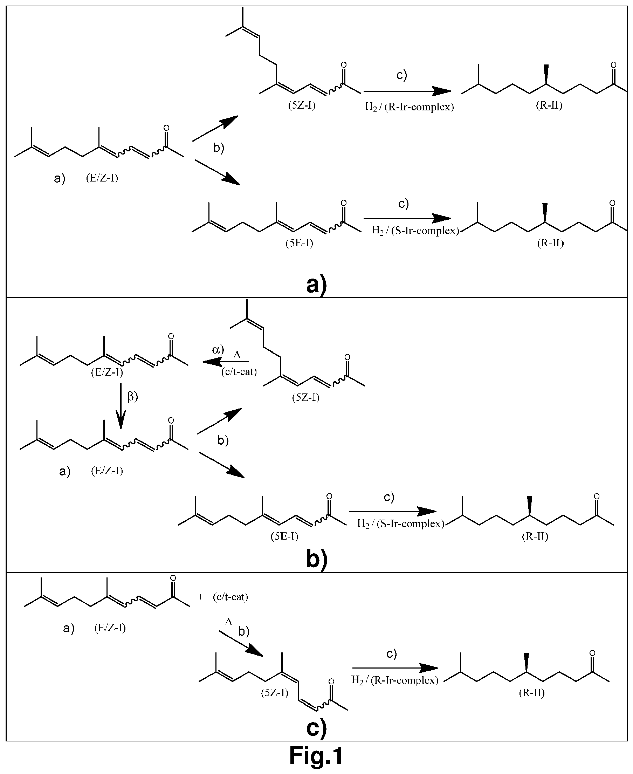

In the following paragraphs some preferred embodiments of the inventions are further discussed by means of schematic figures 1 to 6. This, however, is not to be understood as limiting the invention to the embodiments described here in the figures.

The reference signs in parentheses in the figures, such as (R-V) are used for identification purposes as described below and are not to be confused with the indication of formula such as (V) used in the rest of this document.

The figures 1 to 3 show the subsequent steps 6,10-dimethylundeca-3,5,9-trien-2-one to (6R,10R)-6,10,14-trimethylpentadecan-2-one .

The figures 1 to 4 show the subsequent steps 6,10-dimethylundeca-3,5,9-trien-2-one to (R,R)-isophytol, (2-*ambo*)-*α*-tocopherol and (2R,4'R,8'R)-α-tocopherol, respectively.

In figure 1, three different possibilities for the synthesis of (R)-6,10-dimethylundecan-2-one (R-II) are schematically shown (Figure 1a), 1b), 1c)). As a first step a) for all possibilities shown in figure 1, a mixture of (3E,5E)-/ (3E,5Z)-/ (3Z,5E)-/ and (3Z,5Z)-6,10-dimethylundeca-3,5,9-trien-2-one (*E*/*Z-I*) is provided. In figure 1a) the 5E-isomers, i.e. (3E,5E)- and (3Z,5E)-6,10-dimethylundeca-3,5,9-trien-2-one, (*5E-I*), and the corresponding 5Z-isomers, i.e. (3E,5Z)- and (3Z,5Z)-6,10-dimethylundeca-3,5,9-trien-2-one, (*5Z-I*), are separated in step b) from the mixture provided in step a). The separation in step b) is preferably done by distillation over a column. In step c) the 5Z-isomers are asymmetrically hydrogenated with a specific chiral iridium complex, whereas the E-isomer is asymmetrically hydrogenated with the corresponding enantiomeric chiral iridium complex. A preferred chiral iridium complex is the one of formula (III). The 5E-isomers (*5E*-*I*) are asymmetrically hydrogenated using molecular hydrogen in the presence of the chiral iridium complex of formula (IIIa) (*S-Ir-complex*) having the S-configuration at the stereogenic centre indicated by * in formula (III). The 5Z-isomers (*5Z-I*), on the other hand, are asymmetrically hydrogenated using molecular hydrogen in the presence of the chiral iridium complex of formula (IIIb) (*R-Ir-complex)* having the R-configuration at the stereogenic centre indicated by * in formula (III). Both asymmetric hydrogenation routes furnish the same product, i.e. (R)-6,10-dimethylundecan-2-one (*R-II*)*.* The double bonds at the position 3 and 9 of 6,10-dimethylundeca-3,5,9-trien-2-one are also hydrogenated during the asymmetric hydrogenation. However, as these double bonds are not prochiral, no chiral centres are formed at these positions during the hydrogenation.

In figure 1b) only one of the 5-isomers (5E-isomer (*5E-I*)) (here: desired isomers) are asymmetrically hydrogenated as described above for figure 1a). The other 5-isomers (5Z-isomer (*5Z-I*)) (here: undesired isomers) are subjected to cis/trans isomerization in step α) by addition of a cis/trans isomerization catalyst (*c*/*t-cat*) and heating. The cis/trans isomerization catalyst preferably used is a polythiol, particularly of formula (X). By the action of the cis/trans isomerization catalyst the 5Z-isomers (*5Z-I*)) are isomerized to a mixture of the (3E,5E)-/ (3E,5Z)-/ (3Z,5E)-/ and (3Z,5Z)-6,10-dimethylundeca-3,5,9-trien-2-one (*E*/*Z-I*) which can be added in step β) to the mixture provided in step a). Figure 1b) shows the process in case the 5E-isomers are the desired isomers, i.e. the ones which are asymmetrically hydrogenated. It is obvious that in case the 5Z-isomers are the desired isomers, i.e. the ones which are asymmetrically hydrogenated, the isomerization process would apply in an analogous way to that of the 5E-isomers.

In figure 1c) only one of the 5-isomers (5Z-isomer (*5Z-I*)) (desired isomer) is asymmetrically hydrogenated as described above for figure 1a). A cis/trans isomerization catalyst (*c*/*t-cat*) is added to the mixture of (3E,5E)-/ (3E,5Z)-/ (3Z,5E)-/ and (3Z,5Z)-6,10-dimethylundeca-3,5,9-trien-2-one (*E*/*Z-I*) provided in step a). In step b) the separation of the (desired) isomer (Z-isomer (*Z*-*I*)) is done by distillation in the presence of the cis/trans isomerization catalyst in a (*one-pot isomerization* or *in-situ isomerization*)*.* As the desired isomer is separated by distillation, the remainder, enriched in the higher boiling isomer, is isomerized so that in the distillation vessel a thermodynamic equilibrium between the (3E,5E)-/ (3E,5Z)-/ (3Z,5E)-/ and (3Z,5Z)-isomers is formed continuously. This procedure may allow all of the undesired isomer being present in the isomer mixture at the beginning provided in step a) to be converted to the desired isomer.

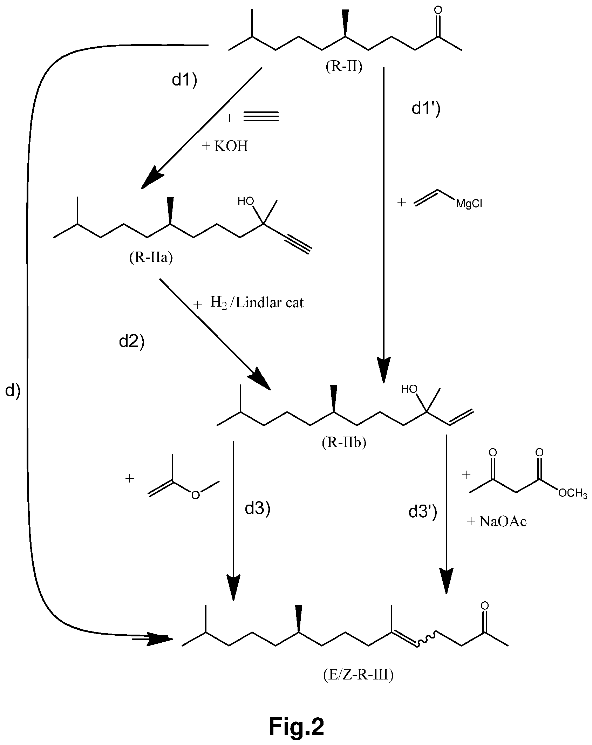

Figure 2 shows the subsequent step d). In step d) (R)-6,10-dimethylundecan-2-one (*R-II*) is chemically transformed to a mixture of (R,E)-6,10.14-trimethylpentadec-5-en-2-one and (R,Z)-6,10,14-trimethylpentadec-5-en-2-one (*E*/*Z-R-III*)*.* Figure 2 also shows the preferred variants of such chemical transformations.

In one of the variants shown in figure 2, (7R)-3,7,11-trimethyldodec-1-en-3-ol (*R-IIb*) is formed from (R)-6,10-dimethylundecan-2-one (*R-II*) by reacting in a first step, i.e. step d1), (R)-6,10-dimethylundecan-2-one (*R-II*) with ethyne (acetylene) in the presence of a base (shown is KOH) to yield the intermediate (7R)-3,7,11-trimethyldodec-1-yn-3-ol (*R-IIa)* and then in second step, i.e. in step d2), reacting with molecular hydrogen in the presence of a Lindlar catalyst.

In another variant (7R)-3,7,11-trimethyldodec-1-en-3-ol (*R-IIb*) is formed directly by means of reaction with a Grignard reagent. In figure 2 vinylmagnesium chloride is shown as Grignard reagent.

Subsequently, two variants for the conversion of (7R)-3,7,11-trimethyldodec-1-en-3-ol (*R-IIb*) to the mixture of (R,E)-6,10,14-trimethylpentadec-5-en-2-one and (R,Z)-6,10,14-trimethylpentadec-5-en-2-one (*E*/*Z-R-III*) are shown in figure 2. In the first variant, (7R)-3,7,11-trimethyldodec-1-en-3-ol (*R-IIb*) is reacted in a Saucy-Marbet reaction (step d3)) with 2-methoxyprop-1-ene to yield a mixture of (R,E)-6,10,14-trimethylpentadec-5-en-2-one and (R,Z)-6,10,14-trimethylpentadec-5-en-2-one (*E*/*Z-R-III*)*.* In the second variant, (7R)-3,7,11-trimethyldodec-1-en-3-ol (*R-IIb*) is reacted with alkyl acetoacetate, preferably methyl acetoacetate, in the presence of a base, preferably sodium acetate, to a mixture of (R.E)-6,10,14-trimethylpentadec-5-en-2-one and (R,Z)-6,10,14-trimethylpentadec-5-en-2-one (*E*/*Z-R-III*) (Carroll rearrangement).

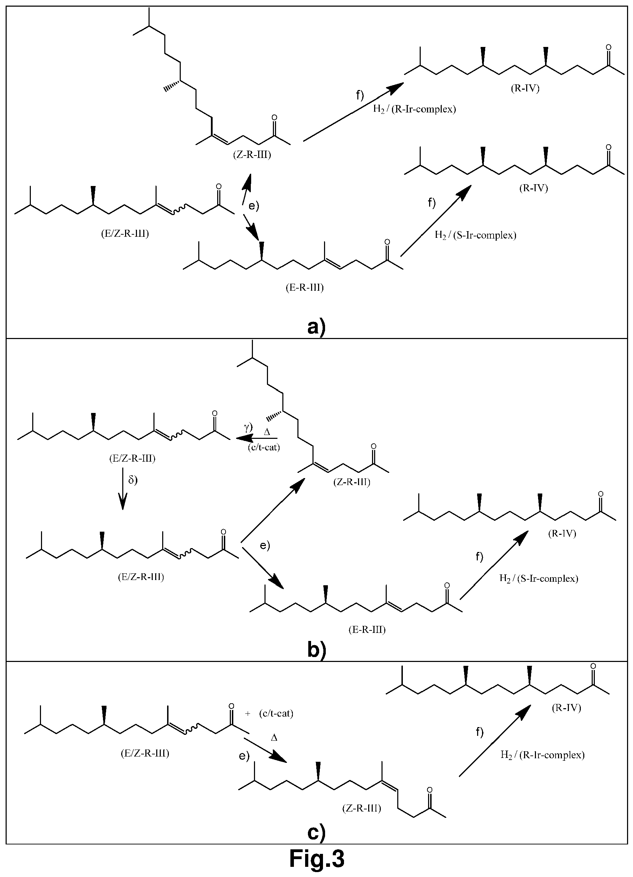

Figure 3 shows the subsequent steps e) and f). Figure 3 corresponds to the figure 1 except that the individual substances are extended by a C5 unit. In analogy, at least one isomer of the mixture of (R,E)-6,10,14-trimethylpentadec-5-en-2-one and (R,Z)-6,10,14-trimethylpentadec-5-en-2-one (*E*/*Z-R-III*) is separated in step e) and asymmetrically hydrogenated to (6R,10R)-6,10,14-trimethylpentadecan-2-one (*R-IV*) in step f).

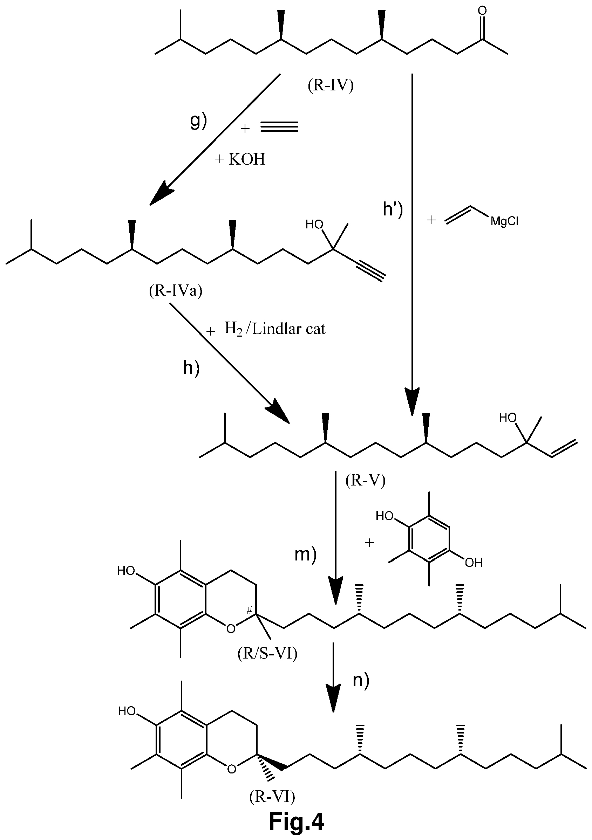

Figure 4 shows the subsequent steps from (6R,10R)-6,10,14-trimethylpentadecan-2-one to (R,R)-isophytol, (*2-ambo*)-α-tocopherol, and (2R,4'R,8'R)-α-tocopherol, respectively.

Figure 4 shows two variants for the conversion of (6R,10R)-6,10,14-trimethylpentadecan-2-one to (R,R)-isophytol. In the first variant, (R,R)-isophytol (*R*-*V*) is formed from (6R,10R)-6,10,14-trimethylpentadecan-2-one (*R-IV*) by reacting in a first step, i.e. step g), (6R,10R)-6,10,14-trimethylpentadecan-2-one (*R*-*IV*) with ethyne (acetylene) in the presence of a base (shown is KOH) to yield the intermediate (7R,11R)-3,7,11,15-tetramethylhexadec-1-yn-3-ol (*R*-*IVa*) and then in second step, i.e. in step h), reacting with molecular hydrogen in the presence of a Lindlar catalyst.

In the other variant shown, (R.R)-isophytol (*R-V*) is formed from (6R,10R)-6,10,14-trimethylpentadecan-2-one (*R-IV*) by means of reaction with a Grignard reagent. In figure 4 vinylmagnesium chloride is shown as Grignard reagent.

(R,R)-isophytol (R-V) can further be condensed in step m) with 2,3,5-trimethylbenzene-1,4-diol to yield (2-*ambo*)-α-tocopherol (*R*/*S-VI*))*.*

In a further step n) (2R,4'R,8'R)-α-tocopherol (*R-VI*)) is isolated from the corresponding (2-*ambo*)-α-tocopherol (*R*/*S-VI*)*.* The isolation is preferably done by chromatographic separation by means of a chiral phase.

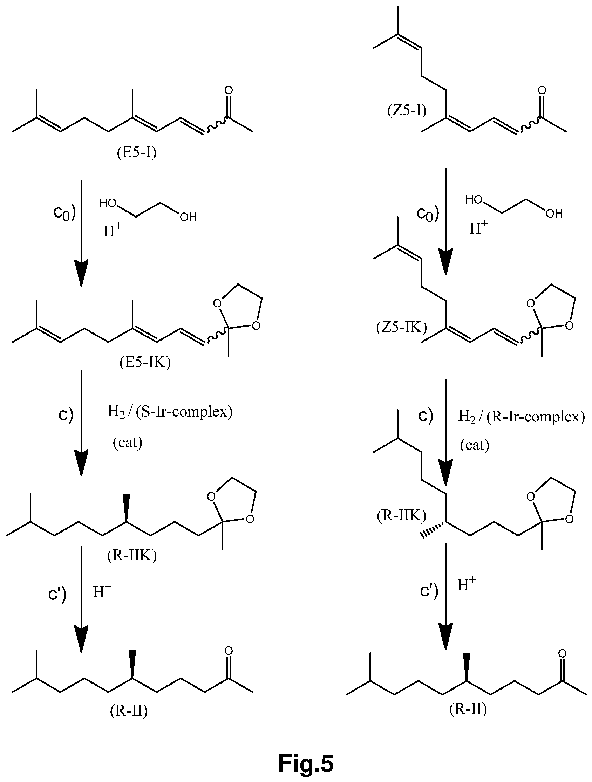

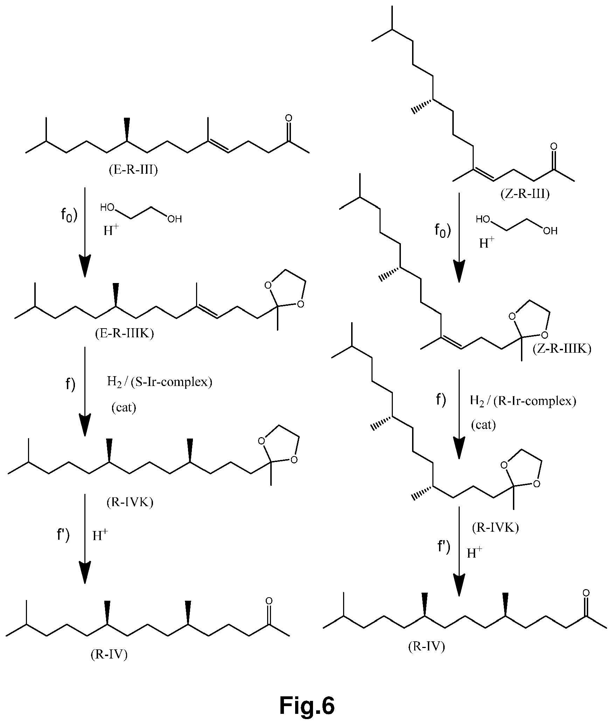

In figures 5 and 6 preferred embodiments of asymmetric hydrogenations are shown. Figure 5 refers to the process steps in figure 1 and figure 6 to the process steps of figure 3.

The left side of figure 5 shows in step c₀) the formation of ketals (*5E-IK*) of the 5E-isomers of (6,10-dimethylundeca-3,5,9-trien-2-one, i.e. (3E,5E)- and (3Z,5E)-6,10-dimethylundeca-3,5,9-trien-2-one, (*5E-I*), which are obtained after separation of the corresponding isomer mixture in step b)) using an alcohol (in figure 5 ethylene glycol is shown) in the presence of an acid. The ketal (*5E-IK*), preferably are then asymmetrically hydrogenated in step c) as discussed in figure 1. The direct product of this asymmetric hydrogenation is an asymmetrically hydrogenated ketal, which after acidic hydrolysis in step c') yields (R)-6,10-dimethylundecan-2-one (*R-II*)*.* On the right side of figure 5 the corresponding reaction scheme is shown for the 5Z-isomers of (6,10-dimethylundeca-3,5,9-trien-2-one, i.e. (3E,5Z)- and (3Z,5Z)-6,10-dimethylundeca-3,5,9-trien-2-one, (*5Z-I*), furnishing via the corresponding ketal intermediate, (*5Z-IK*), respectively, the same compound (R)-6,10-dimethylundecan-2-one (*R-II*).

The left side of figure 6 shows in step fₒ) the formation of ketals (*E-R-IIIK*) of (R,E)-6,10.14-trimethylpentadec-5-en-2-one (*E-R-III*) obtained after isomer separation in step e) using an alcohol (in figure 6 ethylene glycol is shown) in the presence of an acid. The ketal (*E-R-IIIK*), preferably (R,E)-2-methyl-2-(4,8,12-trimethyltridec-3-en-1-yl)-1,3-dioxolane, is then asymmetrically hydrogenated in step f) as discussed in figure 3. The direct product of this asymmetric hydrogenation is an asymmetrically hydrogenated ketal, i.e. 2-methyl-2-((4R,8R)-4,8,12-trimethyltridecyl)-1,3-dioxolane (*R-IVK*), which after acidic hydrolysis in step f') yields (6R,10R)-6,10,14-trimethylpentadecan-2-one (*R-IV*). On the right side of figure 6 the corresponding reaction scheme is shown for the Z-isomer, i.e. (R,Z)-6,10,14-trimethylpentadec-5-en-2-one (*Z-R-III*), furnishing via the ketal intermediate, preferably (Z,E)-2-niethyl-2-(4.8,12-trimethyltridec-3-en-1-yl)-1,3-dioxolane (*Z-R-IIIK*), the same compound (6R,10R)-6,10,14-trimethylpentadecan-2-one (*R-IV*).

## Claims

1. A process of manufacturing (6R,10R)-6,10,14-trimethylpentadecan-2-one in a multistep synthesis from 6,10-dimethylundeca-3,5,9-trien-2-one comprising the steps
a) providing a mixture of (3E,5E)-/ (3E,5Z)-/ (3Z,5E)-/ and (3Z,5Z)-6,10-dimethylundeca-3,5,9-trien-2-one;
b) separating at least one (5E)- and/or at least one (5Z)-isomers of 6,10-dimethylundeca-3,5,9-trien-2-one from the mixture of step a);
c) asymmetric hydrogenation of either 6,10-dimethylundeca-3,5,9-trien-2-one or a ketal of 6,10-dimethylundeca-3,5,9-trien-2-one using molecular hydrogen in the presence of a chiral iridium complex and yielding (R)-6,10-dimethylundecan-2-one ;
d) chemically transforming (R)-6,10-dimethylundecan-2-one to a mixture of (R,E)-6,10,14-trimethylpentadec-5-en-2-one and (R,Z)-6,10,14-trimethylpentadec-5-en-2-one ;
e) separating one isomer of 6,10,14-trimethylpentadec-5-en-2-one from the mixture obtained in step d);
f) asymmetric hydrogenation of either 6,10,14-trimethylpentadec-5-en-2-one or a ketal of 6,10,14-trimethylpentadec-5-en-2-one using molecular hydrogen in the presence of a chiral iridium complex and yielding (6R,10R)-6,10,14-trimethylpentadecan-2-one ;
wherein the steps a)-f) are in the order a,b,c,d,e,f;
with the proviso that
if in the step c) the ketal is hydrogenated
before the step c) a step cₒ) takes place
cₒ) forming a ketal of the isomer of 6,10-dimethylundeca-3,5,9-trien-2-one separated in step b);
and that in step c) the ketal of 6,10-dimethylundeca-3,5,9-trien-2-one is asymmetrically hydrogenated and after the asymmetric hydrogenation the hydrogenated ketal is hydrolysed to the ketone and yielding (R)-6,10-dimethylundecan-2-one;
or
if in the step f) the ketal is hydrogenated
before the step f) a step fₒ) takes place
fₒ) forming a ketal of the isomer of 6,10,14-trimethylpentadec-5-en-2-one separated in step e);
and that in step f) the ketal of 6,10,14-trimethylpentadec-5-en-2-one
is asymmetrically hydrogenated and after the asymmetric hydrogenation the hydrogenated ketal is hydrolysed to the ketone and yielding (6R,10R)-6,10,14-trimethylpentadecan-2-one .

2. The process according to claim 1 **characterized in that** the asymmetric hydrogenation in step c) and/or in step f) takes place in the presence of an additive which is selected from the group consisting of organic sulfonic acids, transition metal salts of organic sulfonic acids, metal alkoxides, aluminoxanes, alkyl aluminoxanes and B(R)₍₃₋ᵥ₎(OZ)ᵥ;
wherein v stands for 0, 1, 2 or 3 and
R stands for F, a C₁₋₆-alkyl, a halogenated C₁₋₆-alkyl, an aryl or halogenated aryl group; and
Z stands for a C₁₋₆-alkyl, a halogenated C₁₋₆-alkyl, an aryl or halogenated aryl group.

3. The process according to any one of the preceding claims **characterized in that** in step d) the chemical transformation of (R)-6,10-dimethylundecan-2-one to a mixture of (R,E)-6,10,14-trimethylpentadec-5-en-2-one and (R,Z)-6,10,14-trimethylpentadec-5-en-2-one is done by the steps
either
d1) ethynylation of (R)-6,10-dimethylundecan-2-one using ethyne in the presence of a basic substance to yield (7R)-3,7,11-trimethyldodec-1-yn-3-ol;
d2) hydrogenation of (7R)-3,7,11-trimethyldodec-1-yn-3-ol with molecular hydrogen in the presence of a Lindlar catalyst to yield (7R)-3,7,11-trimethyldodec-1-en-3-ol;
or
d1')vinylation of (R)-6,10-dimethylundecan-2-one by addition of a vinyl Grignard reagent to yield (7R)-3,7,11-trimethyldodec-1-en-3-ol;
followed by
either
d3) reacting (7R)-3,7,11-trimethyldodec-1-en-3-ol with 2-methoxyprop-1-ene to yield a mixture of (R,E)-6,10,14-trimethylpentadec-5-en-2-one and (R,Z)-6,10,14-trimethylpentadec-5-en-2-one;
or
d3')reacting (7R)-3,7,11-trimethyldodec-1-en-3-ol with an alkyl acetoacetate or diketene in the presence of a base and/or an acid to yield a mixture of (R,E)-6,10,14-trimethylpentadec-5-en-2-one and (R,Z)-6,10,14-trimethylpentadec-5-en-2-one.

4. The process according to any one of the preceding claims **characterized in that** the separation of isomers in step b) and/or e) is done by distillation.

5. The process according to claim 4 **characterized in that** the distillation is done in the presence of a cis/trans isomerization catalyst.

6. The process according to any one of the preceding claims 1-4 **characterized in that** the residual isomer is isomerized by means of a cis/trans isomerization catalyst and added to the corresponding mixture of isomers provided by steps a) resp. d).

7. The process according to any one of the preceding claims **characterized in that** the chiral iridium complex in steps c) and/or f) is a chiral iridium complex of formula (III-0) 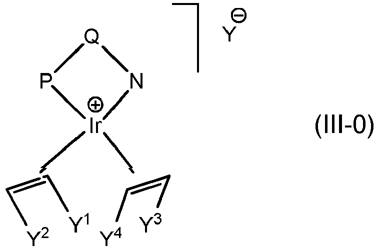 wherein
P-Q-N stands for a chelating organic ligand comprising a stereogenic centre or has planar or axial chirality and has a nitrogen and phosphorous atom as binding site to the iridium centre of the complex;
Y¹, Y², Y³ and Y⁴ independently from each other are hydrogen atoms, C₁₋₁₂-alkyl, C₅₋₁₀-cycloalkyl, or aromatic group; or at least two of them form together at least a two-valent bridged group of at least 2 carbon atoms; and
Y^{⊖} is an anion, particularly selected from the group consisting of halide, PF₆⁻, SbF₆⁻, tetra(3,5-bis(trifluoromethyl)phenyl)borate (BAr_{F}⁻), BF4⁻, perfluorinated sulfonates, preferably F₃C-SO₃⁻ or F₉C₄-SO₃⁻; ClO₄⁻, Al(OC₆F₅)₄⁻, Al(OC(CF₃)₃)₄⁻, N(SO₂CF₃)₂⁻ N(SO₂C4F₉)₂⁻ and B(C₆F₅)₄⁻.

8. The process according to any one of the preceding claims 1-6 **characterized in that** the chiral iridium complex in steps c) and/or f) is a chiral iridium complex of formula (III) 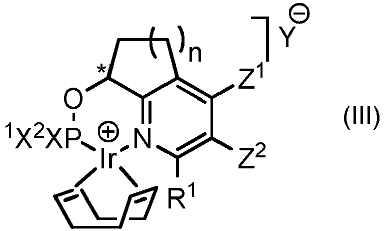 wherein
n is 1 or 2 or 3, preferred 1 or 2;
X¹ and X² are independently from each other hydrogen atoms, C₁₋₄-alkyl, C₅₋₇-cycloalkyl, adamantyl, phenyl (optionally substituted with one to three C₁₋₅-alkyl, C₁₋₄-alkoxy, C₁₋₄-perfluoroalkyl groups and/or one to five halogen atoms)), benzyl, 1-naphthyl, 2-naphthyl, 2-furyl or ferrocenyl;
Z¹ and Z² are independently from each other hydrogen atoms, C₁₋₅-alkyl or C₁₋₅-alkoxy groups;
or Z¹ and Z² stand together for a bridging group forming a 5 or 6 membered ring;
Y^{⊝} is an anion, particularly selected from the group consisting of halide, PF₆⁻, SbF₆⁻, tetra(3,5-bis(trifluoromethyl)phenyl)borate(BAr_{F}⁻), BF₄⁻, perfluorinated sulfonates, preferably F₃C-SO₃⁻ or F₉C₄-SO₃⁻ ; ClO₄⁻, Al(OC₆F₅)₄⁻, Al(OC(CF₃)₃)₄⁻, N(SO₂CF₃)₂⁻ N(SO₂C₄F₉)₂⁻ and B(C₆F₅)₄⁻;
R¹ represents either phenyl or o-tolyl or m-tolyl or p-tolyl or a group of formula (IVa) or (IVb) or (IVc) 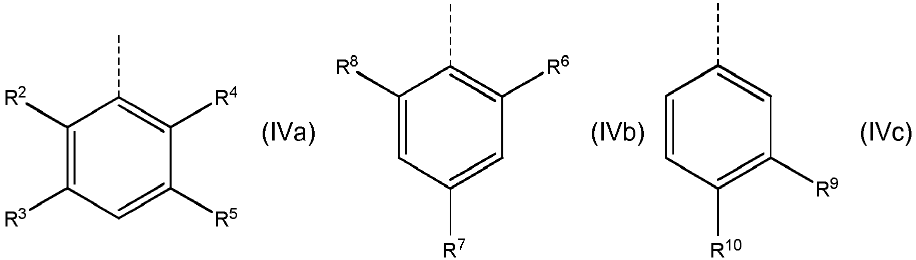
wherein R² and R³ represent either both H or a C₁-C₄-alkyl group or a halogenated C₁-C₄-alkyl group or represent a divalent group forming together a 6-membered cycloaliphatic or an aromatic ring which optionally is substituted by halogens atoms or by C₁-C₄-alkyl groups or by C₁-C₄-alkoxy groups
R⁴ and R⁵ represent either both H or a C₁-C₄-alkyl group or a halogenated C₁-C₄-alkyl group or a divalent group forming together a 6-membered cycloaliphatic or an aromatic ring which optionally is substituted by halogens atoms or by C₁-C₄-alkyl groups or by C₁-C₄-alkoxy groups;
R⁶ and R⁷ and R⁸ represent each a C₁-C₄-alkyl group or a halogenated C₁-C₄-alkyl group;
R⁹ and R¹⁰ represent either both H or a C₁-C₄-alkyl group or a halogenated C₁-C₄-alkyl group or a divalent group forming together a 6-membered cycloaliphatic or an aromatic ring which optionally is substituted by halogens atoms or by C₁-C₄-alkyl groups or by C₁-C₄-alkoxy groups;
and wherein * represents a stereogenic centre of the complex of formula (III).

9. The process according to claim 8 **characterized in that** the chiral iridium complex of formula (III) used in step c) and/or f) used for the asymmetric hydrogenation has the
S-configuration at the stereogenic centre indicated by * in case (3E,5E)-or (3Z,5E)-6,10-dimethylundeca-3,5,9-trien-2-one or ketals thereof, or (R,E)-6,10,14-trimethylpentadec-5-en-2-one, or a ketal thereof, are to be hydrogenated;
or has the
R-configuration at the stereogenic centre indicated by * in case (3E,5Z)-or (3Z,5Z)-6,10-dimethylundeca-3,5,9-trien-2-one or ketals thereof, or (R,Z)-6,10,14-trimethylpentadec-5-en-2-one, or a ketal thereof, are to be hydrogenated.

10. A process of manufacturing (R,R)-isophytol ((3RS,7R,11R)-3,7,11,15-tetramethylhexadec-1-en-3-ol) comprising
a process of manufacturing (6R,10R)-6,10,14-trimethylpentadecan-2-one as described in the process according any one of the preceding claims 1-9; followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol.

11. A process of manufacturing compound of formula (V) comprising a process of manufacturing (6R,10R)-6,10,14-trimethylpentadecan-2-one as described in the process according any one of the preceding claims 1-9; followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol;
followed by the steps
m) condensing (R,R)-isophytol with compound of formula (VI) to yield compound of formula (V) being an isomeric mixture in view of the chirality at the centre indicated by #; 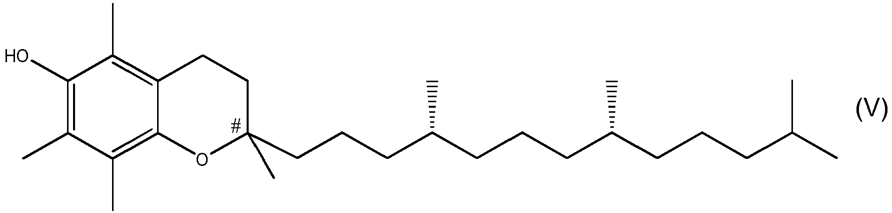 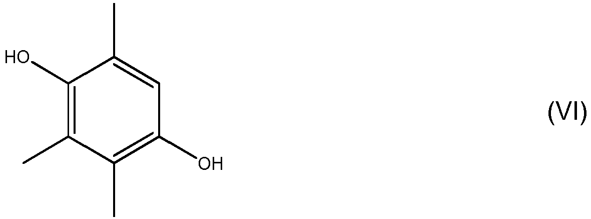
wherein # represents a stereogenic centre.

12. A process of manufacturing compound of formula (V-A) comprising a process of manufacturing (6R,10R)-6,10,14-trimethylpentadecan-2-one as described in the process according any one of the preceding claims 1-9; followed by the steps
either
g) ethynylation of (6R,10R)-6,10,14-trimethylpentadecan-2-one using ethyne in the presence of a basic substance to yield (7R,11R)-3,7,11,15-tetramethylhexadec-1-yn-3-ol;
h) hydrogenation of (7R,11R)-3,7,11,15-tetramethylhexadec-1-yn-3-ol with molecular hydrogen in the presence of a Lindlar catalyst to yield (R,R)-isophytol;
or
h') vinylation of (6R,10R)-6,10,14-trimethylpentadecan-2-one by addition of a vinyl Grignard reagent to yield (R,R)-isophytol;
followed by the steps
m) condensing (R,R)-isophytol with compound of formula (VI) to yield compound of formula (V) being an isomeric mixture in view of the chirality at the centre indicated by #; 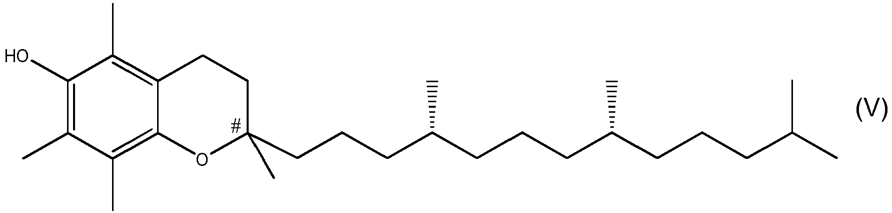 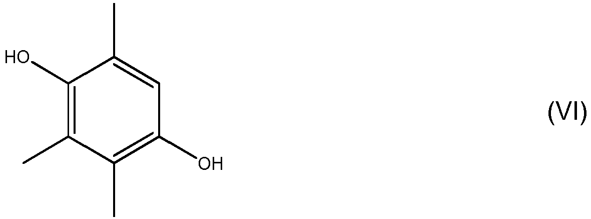
wherein # represents a stereogenic centre; and
n) isolating compound of formula (V-A) from the isomeric mixture of formula (V)

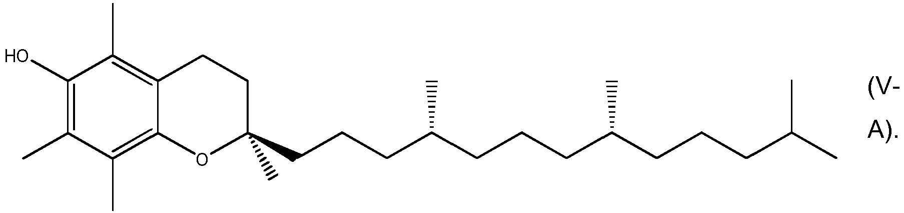

13. Composition comprising
- at least one ketal of formula (XI) and
- at least one chiral iridium complex 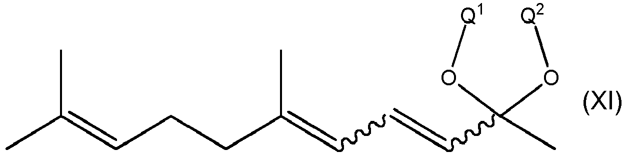 wherein a wavy line represents a carbon-carbon bond which is linked to the adjacent carbon-carbon double bond so as to have said carbon-carbon double bond either in the Z or in the E-configuration;
and wherein
Q¹ and Q²
stand either individually or both for a C₁-C₁₀ alkyl group or a halogenated C₁-C₁₀ alkyl group;
or form together a C₂-C₆ alkylene group or a C₆-C₈ cycloalkylene group.

14. Ketal which is selected from the group consisting of (R)-6,10-dimethyl-2,2-bis(2,2,2-trifluoroethoxy)undecane, 2,5,5-trimethyl-2-((4R,8R)-4,8,12-trimethyltridecyl)-1,3-dioxane and (6R,10R)-6,10,14-trimethyl-2,2-bis(2,2,2-trifluoroethoxy)pentadecane.

## Patentansprüche

1. Verfahren zur Herstellung von (6R,10R)-6,10,14-Trimethylpentadecan-2-on in einer Mehrschrittsynthese aus 6,10-Dimethylundeca-3,5,9-trien-2-on, das folgende Schritte umfasst:
a) Bereitstellen eines Gemischs von (3E,5E)-, (3E,5Z)-, (3Z,5E)- und (3Z,5Z)-6,10-Dimethylundeca-3,5,9-trien-2-on;
b) Abtrennen mindestens eines (5E)- und/oder mindestens eines (5Z)-Isomers von 6,10-Dimethylundeca-3,5,9-trien-2-on aus dem Gemisch von Schritt a);
c) asymmetrische Hydrierung von 6,10-Dimethylundeca-3, 5, 9-trien-2-on oder einem Ketal von 6,10-Dimethylundeca-3,5,9-trien-2-on mit molekularem Wasserstoff in Gegenwart eines chiralen Iridiumkomplexes unter Erhalt von (R)-6,10-Dimethylundecan-2-on;
d) chemisches Umwandeln von (R)-6,10-Dimethylundecan-2-on in ein Gemisch von (R,E)-6, 10, 14-Trimethylpentadec-5-en-2-on und (R,Z)-6,10,14-Trimethylpentadec-5-en-2-on;
e) Abtrennen eines Isomers von 6,10,14-Trimethylpentadec-5-en-2-on aus dem in Schritt d) erhaltenen Gemisch;
f) asymmetrische Hydrierung von 6,10,14-Trimethylpentadec-5-en-2-on oder einem Ketal von 6,10,14-Trimethylpentadec-5-en-2-on mit molekularem Wasserstoff in Gegenwart eines chiralen Iridiumkomplexes unter Erhalt von (6R,10R)-6,10,14-Trimethylpentadecan-2-on;
wobei die Schritte a) - f) in der Reihenfolge a, b, c, d, e, f durchgeführt werden;
mit der Maßgabe, dass
dann, wenn in Schritt c) das Ketal hydriert wird,
vor Schritt c) ein Schritt cₒ) stattfindet:
cₒ) Bilden eines Ketals des in Schritt b) abgetrennten Isomers von 6,10-Dimethylundeca-3,5,9-trien-2-on;
und dass in Schritt c) das Ketal von 6,10-Dimethylundeca-3,5,9-trien-2-on asymmetrisch hydriert wird und das hydrierte Ketal nach der asymmetrischen Hydrierung zum Keton hydrolysiert wird, was (R)-6,10-Dimethylundecan-2-on ergibt;
oder
dann, wenn in Schritt f) das Ketal hydriert wird,
vor Schritt f) ein Schritt fₒ) stattfindet:
fₒ) Bilden eines Ketals des in Schritt e) abgetrennten Isomers von 6,10,14-Trimethylpentadec-5-en-2-on;
und dass in Schritt f) das Ketal von 6,10,14-Trimethylpentadec-5-en-2-on asymmetrisch hydriert wird und das hydrierte Ketal nach der asymmetrischen Hydrierung zum Keton hydrolysiert wird, was (6R,10R)-6,10,14-Trimethylpentadecan-2-on ergibt.

2. Verfahren nach Anspruch 1, **dadurch gekennzeichnet, dass** die asymmetrische Hydrierung in Schritt c) und/oder in Schritt f) in Gegenwart eines Additivs aus der Gruppe bestehend aus organischen Sulfonsäuren, Übergangsmetallsalzen organischer Sulfonsäuren, Metallalkoxiden, Aluminoxanen, Alkylaluminoxanen und B(R)_{(3-v}}(OZ)ᵥ stattfindet;
wobei v für 0, 1, 2 oder 3 steht und
R für F, eine C₁₋₆-Alkylgruppe, eine halogenierte C₁₋₆-Alkylgruppe, eine Arylgruppe oder eine halogenierte Arylgruppe steht; und
Z für eine C₁₋₆-Alkylgruppe, eine halogenierte C₁₋₆-Alkylgruppe, eine Arylgruppe oder eine halogenierte Arylgruppe steht.

3. Verfahren nach einem der vorhergehenden Ansprüche, **dadurch gekennzeichnet, dass** in Schritt d) die chemische Umwandlung von (R)-6,10-Dimethylundecan-2-on in ein Gemisch von (R,E)-6,10,14-Trimethylpentadec-5-en-2-on und (R,Z)-6,10,14-Trimethylpentadec-5-en-2-on durch folgende Schritte erfolgt:
entweder
d1) Ethinylierung von (R)-6,10-Dimethylundecan-2-on mit Ethin in Gegenwart einer basischen Substanz zu (7R)-3,7,11-Trimethyldodec-1-in-3-ol;
d2) Hydrierung von (7R)-3,7,11-Trimethyldodec-1-in-3-ol mit molekularem Wasserstoff in Gegenwart eines Lindlar-Katalysators zu (7R)-3,7,11-Trimethyldodec-1-en-3-ol;
oder
d1') Vinylierung von (R)-6,10-Dimethylundecan-2-on durch Zugabe eines Vinyl-Grignard-Reagens zu (7R)-3,7,11-Trimethyldodec-1-en-3-ol;
gefolgt von
entweder
d3) Umsetzen von (7R)-3,7,11-Trimethyldodec-1-en-3-ol mit 2-Methoxyprop-1-en zu einem Gemisch von (R,E)-6,10,14-Trimethylpentadec-5-en-2-on und (R,Z)-6, 10, 14-trimethylpentadec-5-en-2-on;
oder
d3') Umsetzen von (7R)-3,7,11-Trimethyldodec-1-en-3-ol mit einem Acetessigsäurealkylester oder Diketen in Gegenwart einer Base und/oder einer Säure zu einem Gemisch von (R,E)-6,10,14-Trimethylpentadec-5-en-2-on und (R,Z)-6,10,14-Trimethylpentadec-5-en-2-on.

4. Verfahren nach einem der vorhergehenden Ansprüche, **dadurch gekennzeichnet, dass** die Abtrennung von Isomeren in Schritt b) und/oder e) durch Destillation erfolgt.

5. Verfahren nach Anspruch 4, **dadurch gekennzeichnet, dass** die Destillation in Gegenwart eines cis/trans-Isomerisierungskatalysators durchgeführt wird.

6. Verfahren nach einem der Ansprüche 1-4, **dadurch gekennzeichnet, dass** das verbliebene Isomer mit Hilfe eines cis/trans-Isomerisierungskatalysators isomerisiert und zu dem entsprechenden, durch die Schritte a) bzw. d) bereitgestellten Isomerengemisch gegeben wird.

7. Verfahren nach einem der vorhergehenden Ansprüche, **dadurch gekennzeichnet, dass** es sich bei dem chiralen Iridiumkomplex in den Schritten c) und/oder f) um einen chiralen Iridiumkomplex der Formel (III-0) 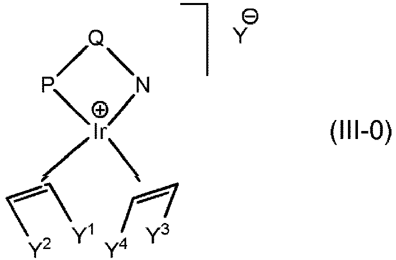 handelt, wobei
P-Q-N für einen chelatbildenden organischen Liganden steht, der ein stereogenes Zentrum oder planare oder axiale Chiralität aufweist und ein Stickstoff- und Phosphoratom als Bindungsstelle zum Iridiumzentrum des Komplexes aufweist;
Y¹, Y², Y³ und Y⁴ unabhängig voneinander für Wasserstoffatome, eine C₁₋₁₂-Alkylgruppe, eine C₅₋₁₀-Cycloalkylgruppe oder eine aromatische Gruppe stehen oder mindestens zwei davon zusammen mindestens eine zweiwertige verbrückte Gruppe mit mindestens 2 Kohlenstoffatomen bilden; und
Y^{⊝} für ein Anion steht, insbesondere ausgewählt aus der Gruppe bestehend aus Halogenid, PF₆⁻, SbF₆⁻, Tetra(3,5-bis(trifluormethyl)phenyl)borat (BAr_{F}⁻), BF4⁻, perfluorierten Sulfonaten, bevorzugt F₃C-SO₃⁻ oder F₉C₄-SO₃⁻; ClO₄⁻, Al(OC₆F₅)₄⁻, Al(OC(CF₃)₃)₄⁻, N(SO₂CF₃)₂⁻N(SO₂C₄F₉)₂⁻ und B(C₆F₅)₄⁻.

8. Verfahren nach einem der vorhergehenden Ansprüche 1-6, **dadurch gekennzeichnet, dass** es sich bei dem chiralen Iridiumkomplex in den Schritten c) und/oder f) um einen chiralen Iridiumkomplex der Formel (III) 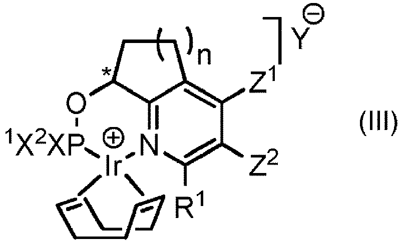 handelt, wobei
n für 1 oder 2 oder 3, bevorzugt 1 oder 2, steht; X¹ und X² unabhängig voneinander für Wasserstoffatome, C₁₋₄-Alkyl, C₅₋₇-Cycloalkyl, Adamantyl, Phenyl (gegebenenfalls substituiert durch eine bis drei C₁₋₅-Alkyl-, C₁₋₄-Alkoxy-, C₁₋₄-Perfluoralkylgruppen und/oder ein bis fünf Halogenatome), Benzyl, 1-Naphthyl, 2-Naphthyl, 2-Furyl oder Ferrocenyl stehen;
Z¹ und Z² unabhängig voneinander für Wasserstoffatome, C₁₋₅-Alkyl- oder C₁₋₅-Alkoxygruppen stehen;
oder Z¹ und Z² zusammen für eine Brückengruppe stehen, die einen 5- bis 6-gliedrigen Ring bildet;
Y^{Θ} für ein Anion steht, insbesondere ausgewählt aus der Gruppe bestehend aus Halogenid, PF₆⁻, SbF₆⁻, Tetra(3,5-bis(trifluormethyl)henyl)borat(BAr_{F}⁻), BF4⁻, perfluorierten Sulfonaten, vorzugsweise F₃C-SO₃⁻ oder F₉C₄-SO₃⁻; ClO₄⁻, Al(OC₆F₅)₄⁻, Al(OC(CF₃)₃)₄⁻, N(SO₂CF₃)₂⁻, N(SO₂C₄F₉)₂⁻ und B(C₆F₅)₄⁻;
R¹ entweder für Phenyl oder o-Tolyl oder m-Tolyl oder p-Tolyl oder eine Gruppe der Formel (IVa) oder (IVb) oder (IVc) 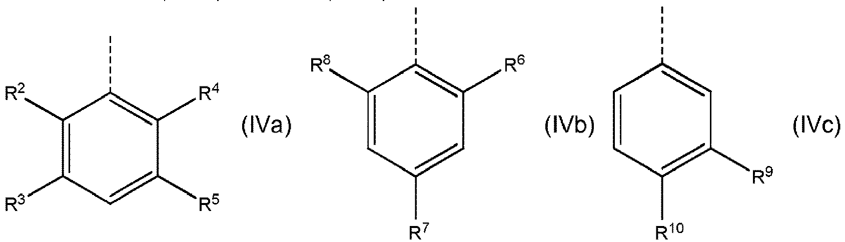 steht, wobei R² und R³ entweder beide für H oder eine C₁-C₄-Alkylgruppe oder eine halogenierte C₁-C₄-Alkylgruppe stehen oder für eine zweiwertige Gruppe, die zusammen einen 6-gliedrigen cycloaliphatischen oder aromatischen Ring bildet, der gegebenenfalls durch Halogenatome oder durch C₁-C₄-Alkylgruppen oder durch C₁-C₄-Alkoxygruppen substituiert ist, stehen;
R⁴ und R⁵ entweder beide für H oder eine C₁-C₄-Alkylgruppe oder eine halogenierte C₁-C₄-Alkylgruppe oder für eine zweiwertige Gruppe, die zusammen einen 6-gliedrigen cycloaliphatischen oder aromatischen Ring bildet, der gegebenenfalls durch Halogenatome oder durch C₁-C₄-Alkylgruppen oder durch C₁-C₄-Alkoxygruppen substituiert ist, stehen;
R⁶ und R⁷ und R⁸ jeweils für eine C₁-C₄-Alkylgruppe oder eine halogenierte C₁-C₄-Alkylgruppe stehen;
R⁹ und R¹⁰ entweder beide für H oder eine C₁-C₄-Alkylgruppe oder eine halogenierte C₁-C₄-Alkylgruppe stehen oder für eine zweiwertige Gruppe, die zusammen einen 6-gliedrigen cycloaliphatischen oder aromatischen Ring bildet, der gegebenenfalls durch Halogenatome oder durch C₁-C₄-Alkylgruppen oder durch C₁-C₄-Alkoxygruppen substituiert ist, stehen;
und wobei * ein stereogenes Zentrum des Komplexes der Formel (III) darstellt.

9. Verfahren nach Anspruch 8, **dadurch gekennzeichnet, dass** der chirale Iridiumkomplex der Formel (III), der in Schritt c) und/oder f) verwendet wird und der für die asymmetrische Hydrierung verwendet wird, die
S-Konfiguration an dem mit * markierten stereogenen Zentrum aufweist, wenn (3E,5E)-oder (3Z,5E)-6,10-Dimethylundeca-3,5,9-trien-2-on oder Ketale davon bzw. (R,E)-6,10,14-Trimethylpentadec-5-en-2-on oder ein Ketal davon zu hydrieren sind;
oder die
R-Konfiguration an dem mit * markierten stereogenen Zentrum aufweist, wenn (3E,5Z)-oder (3Z,5Z)-6,10-Dimethylundeca-3,5,9-trien-2-on oder Ketale davon bzw. (R,Z)-6,10,14-Trimethylpentadec-5-en-2-on oder ein Ketal davon zu hydrieren sind.

10. Verfahren zur Herstellung von (R,R)-Isophytol ((3RS,7R,11R)-3,7,11,15-Tetramethylhexadec-1-en-3-ol), das Folgendes umfasst:
ein Verfahren zur Herstellung von (6R,10R)-6,10,14-Trimethylpentadecan-2-on, wie in dem Verfahren nach einem der vorhergehenden Ansprüche 1-9 beschrieben;
gefolgt von den folgenden Schritten:
entweder
g) Ethinylierung von (6R,10R)-6,10,14-Trimethylpentadecan-2-on mit Ethin in Gegenwart einer basischen Substanz zu (7R,11R)-3,7,11,15-Tetramethylhexadec-1-in-3-ol;
h) Hydrierung von (7R,11R)-3,7,11,15-Tetramethylhexadec-1-in-3-ol mit molekularem Wasserstoff in Gegenwart eines Lindlar-Katalysators zu (R,R)-Isophytol;
oder
h') Vinylierung von (6R,10R)-6,10,14-Trimethylpentadecan-2-on durch Zugabe eines Vinyl-Grignard-Reagens zu (R,R)-Isophytol.

11. Verfahren zur Herstellung der Verbindung der Formel (V), das Folgendes umfasst:
ein Verfahren zur Herstellung von (6R,10R)-6, 10, 14-Trimethylpentadecan-2-on, wie in dem Verfahren nach einem der vorhergehenden Ansprüche 1-9 beschrieben;
gefolgt von den folgenden Schritten:
entweder
g) Ethinylierung von (6R,10R)-6,10,14-Trimethylpentadecan-2-on mit Ethin in Gegenwart einer basischen Substanz zu (7R,11R)-3,7,11,15-Tetramethylhexadec-1-in-3-ol;
h) Hydrierung von (7R,11R)-3,7,11,15-Tetramethylhexadec-1-in-3-ol mit molekularem Wasserstoff in Gegenwart eines Lindlar-Katalysators zu (R,R)-Isophytol;
oder
h') Vinylierung von (6R,10R)-6,10,14-Trimethylpentadecan-2-on durch Zugabe eines Vinyl-Grignard-Reagens zu (R,R)-Isophytol;
gefolgt von den folgenden Schritten:
m) Kondensieren von (R,R)-Isophytol mit der Verbindung der Formel (VI) zu der Verbindung der Formel (V), bei der es sich im Hinblick auf die Chiralität am durch # markierten Zentrum um ein Isomerengemisch handelt; 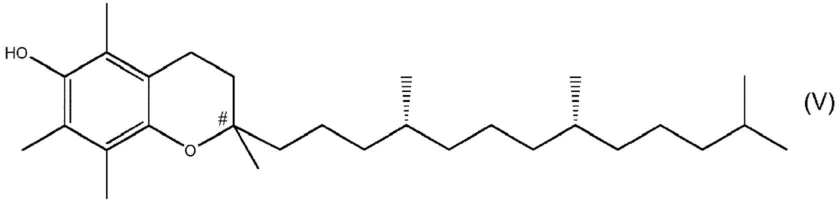 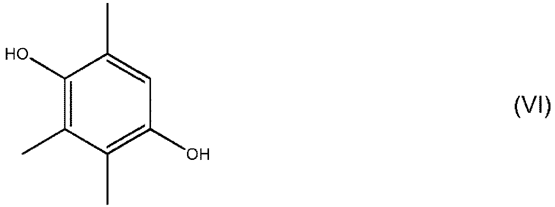
wobei # ein stereogenes Zentrum darstellt.

12. Verfahren zur Herstellung der Verbindung der Formel (V-A), das Folgendes umfasst:
ein Verfahren zur Herstellung von (6R,10R)-6, 10, 14-Trimethylpentadecan-2-on, wie in dem Verfahren nach einem der vorhergehenden Ansprüche 1-9 beschrieben;
gefolgt von den folgenden Schritten:
entweder
g) Ethinylierung von (6R,10R)-6,10,14-Trimethylpentadecan-2-on mit Ethin in Gegenwart einer basischen Substanz zu (7R,11R)-3,7,11,15-Tetramethylhexadec-1-in-3-ol;
h) Hydrierung von (7R,11R)-3,7,11,15-Tetramethylhexadec-1-in-3-ol mit molekularem Wasserstoff in Gegenwart eines Lindlar-Katalysators zu (R,R)-Isophytol;
oder
h') Vinylierung von (6R,10R)-6,10,14-Trimethylpentadecan-2-on durch Zugabe eines Vinyl-Grignard-Reagens zu (R,R)-Isophytol;
gefolgt von den folgenden Schritten:
m) Kondensieren von (R,R)-Isophytol mit der Verbindung der Formel (VI) zu der Verbindung der Formel (V), bei der es sich im Hinblick auf die Chiralität am durch # markierten Zentrum um ein Isomerengemisch handelt; 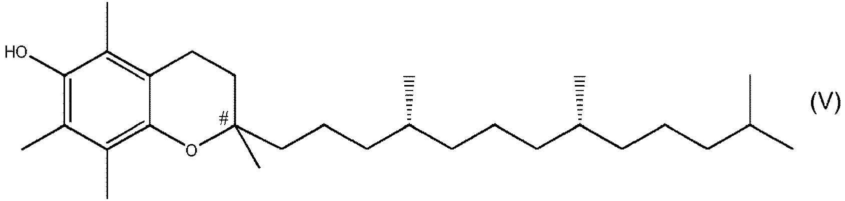 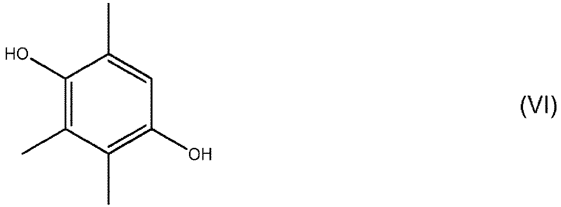
wobei # ein stereogenes Zentrum darstellt; und n) Isolieren der Verbindung der Formel (V-A) aus dem Isomerengemisch der Formel (V)

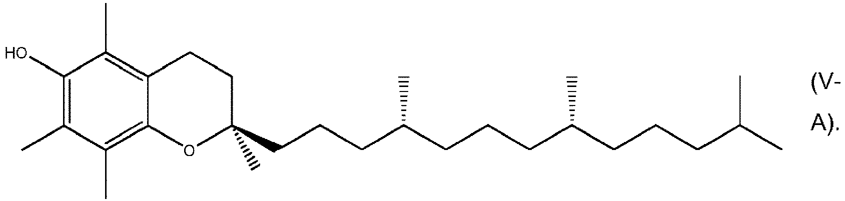

13. Zusammensetzung, umfassend
- mindestens ein Ketal der Formel (XI) und
- mindestens einen chiralen Iridiumkomplex 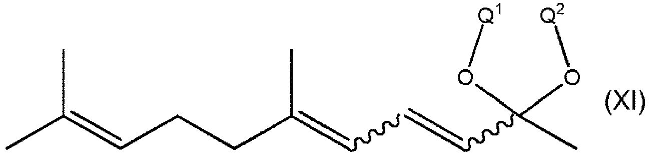 wobei eine Wellenlinie eine Kohlenstoff-Kohlenstoff-Bindung darstellt, die so mit der benachbarten Kohlenstoff-Kohlenstoff-Doppelbindung verknüpft ist, dass die Kohlenstoff-Kohlenstoff-Doppelbindung entweder in der Z-oder in der E-Konfiguration vorliegt;
und wobei
Q¹ und Q²
entweder einzeln oder beide für eine C₁₋₁₀-Alkylgruppe oder eine halogenierte C₁₋₁₀-Alkylgruppe stehen
oder zusammen eine C₂₋₆-Alkylengruppe oder eine C₆₋₈-Cycloalkylengruppe bilden.

14. Ketal, das ausgewählt ist aus der Gruppe bestehend aus (R)-6,10-Dimethyl-2,2-bis(2,2,2-trifluorethoxy)undecan, 2,5,5-Trimethyl-2-((4R,8R)-4,8,12-trimethyltridecyl)-1,3-dioxan und (6R,10R)-6,10,14-Trimethyl-2,2-bis(2,2,2-trifluorethoxy)pentadecan.

## Revendications

1. Procédé de fabrication de (6R,10R)-6,10,14-triméthylpentadécan-2-one dans une synthèse à étapes multiples à partir de 6,10-diméthylundéca-3,5,9-trién-2-one comprenant les étapes de
a) fourniture d'un mélange de (3E,5E)-/ (3E,5Z)-/ (3Z,5E)-/ et (3Z, 5Z)-6, 10- diméthylundéca-3,5,9-trién-2-one ;
b) séparation d'au moins un isomère (5E) et/ou au moins un isomère (5Z) de 6,10-diméthylundéca-3,5,9-trién-2-one à partir du mélange de l'étape a) ;
c) hydrogénation asymétrique de 6,10-diméthylundéca-3,5,9-trién-2-one ou d'un cétal de 6,10-diméthylundéca-3,5,9-trién-2-one en utilisant de l'hydrogène moléculaire en présence d'un complexe d'iridium chiral et obtention de (R)-6,10-diméthylundécan-2-one ;
d) transformation chimique de (R)-6,10-diméthylundécan-2-one en un mélange de (R,E)-6,10,14-triméthylpentadéc-5-én-2-one et de (R,Z)-6,10,14-triméthyl-pentadéc-5-én-2-one ;
e) séparation d'un isomère de 6,10,14-triméthylpentadéc-5-én-2-one à partir du mélange obtenu dans l'étape d) ;
f) hydrogénation asymétrique de 6,10,14-triméthylpentadéc-5-én-2-one ou d'un cétal de 6,10,14-triméthylpentadéc-5-én-2-one en utilisant de l'hydrogène moléculaire en présence d'un complexe d'iridium chiral et obtention de (6R,10R)-6,10,14-triméthylpentadécan-2-one ; dans lequel les étapes a) à f) sont dans l'ordre a, b, c, d, e, f ;
à condition que
si, dans l'étape c), le cétal est hydrogéné avant l'étape c), une étape cₒ) est conduite
cₒ) formation d'un cétal de l'isomère de 6,10-diméthylundéca-3,5,9-trién-2-one séparé dans l'étape b) ;
et que, dans l'étape c), le cétal de 6,10-diméthylundéca-3,5,9-trién-2-one est hydrogéné de façon asymétrique et, après l'hydrogénation asymétrique, le cétal hydrogéné est hydrolysé en cétone et obtention de (R)-6,10-diméthylundécan-2-one ;
ou
si dans l'étape f), le cétal est hydrogéné avant l'étape f), une étape fₒ) est conduite
fₒ) formation d'un cétal de l'isomère de 6,10,14-triméthylpentadéc-5-én-2-one séparé dans l'étape e) ; et que, dans l'étape f), le cétal de 6,10,14-triméthylpentadéc-5-én-2-one est hydrogéné de façon asymétrique et, après l'hydrogénation asymétrique, le cétal hydrogéné est hydrolysé en cétone et obtention de (6R,10R)-6,10,14-triméthylpentadécan-2-one.

2. Procédé selon la revendication 1 **caractérisé en ce que** l'hydrogénation asymétrique dans l'étape c) et/ou dans l'étape f) est conduite en présence d'un additif qui est choisi dans le groupe constitué d'acides sulfoniques organiques, sels de métal de transition d'acides sulfoniques organiques, alcoxydes métalliques, aluminoxanes, alkylaluminoxanes et B (R)₍₃₋ᵥ₎(OZ)ᵥ ; où v désigne 0, 1, 2 ou 3 et
R désigne F, un groupe alkyle en C₁₋₆, alkyle en C₁₋₆ halogéné, aryle ou aryle halogéné ; et
Z désigne un groupe alkyle en C₁₋₆, alkyle en C₁₋₆ halogéné, aryle ou aryle halogéné.

3. Procédé selon l'une quelconque des revendications précédentes **caractérisé en ce que** dans l'étape d), la transformation chimique de la (R)-6,10-diméthylundécan-2-one en un mélange de (R,E)-6,10,14-triméthylpentadéc-5-én-2-one et de (R,Z)-6,10,14-triméthylpentadéc-5-én-2-one est effectuée par l'une des étapes de
d1) éthynylation de (R)-6,10-diméthylundécan-2-one en utilisant de l'éthyne en présence d'une substance basique pour obtenir le (7R)-3,7,11-triméthyldodéc-1-yn-3-ol ; d2) hydrogénation de (7R)-3,7,11-triméthyldodéc-1-yn-3-ol avec de l'hydrogène moléculaire en présence d'un catalyseur de Lindlar pour obtenir le (7R)-3,7,11-triméthyldodéc-1-én-3-ol ; ou
d1') vinylation de la (R)-6,10-diméthylundécan-2-one par ajout d'un réactif de Grignard vinylique pour obtenir le (7R)-3,7,11-triméthyldodéc-1-én-3-ol ;
suivie par l'une des étapes de
d3) réaction de (7R)-3,7,11-triméthyldodéc-1-én-3-ol avec le 2-méthoxyprop-1-ène pour obtenir un mélange de (R,E)-6,10,14-triméthylpentadéc-5-én-2-one et de (R,Z)-6,10,14-triméthylpentadéc-5-én-2-one ;
ou
d3') réaction de (7R)-3,7,11-triméthyldodéc-1-én-3-ol avec un acétoacétate d'alkyle ou du dicétène en présence d'une base et/ou un acide pour obtenir un mélange de (R,E)-6,10,14-triméthylpentadéc-5-én-2-one et de (R,Z)-6, 10, 14-triméthyle pentadéc-5-én-2-one.

4. Procédé selon l'une quelconque des revendications précédentes **caractérisé en ce que** la séparation d'isomères dans l'étape b) et/ou e) est effectuée par distillation.

5. Procédé selon la revendication 4 **caractérisé en ce que** la distillation est effectuée en présence d'un catalyseur d'isomérisation cis/trans.

6. Procédé selon l'une quelconque des revendications précédentes 1 à 4 **caractérisé en ce que** l'isomère résiduel est isomérisé au moyen d'un catalyseur d'isomérisation cis/trans et ajouté au mélange d'isomères correspondant produit par les étapes a) et respectivement d) .

7. Procédé selon l'une quelconque des revendications précédentes **caractérisé en ce que** le complexe d'iridium chiral dans les étapes c) et/ou f) est un complexe d'iridium chiral de formule (III-0) 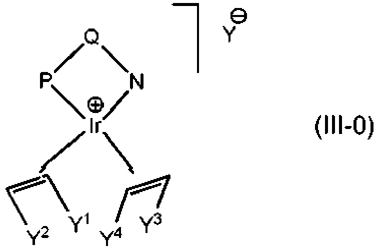 dans lequel
P-Q-N désigne un ligand organique chélateur comprenant un centre stéréogénique ou présente une chiralité plane ou axiale et a un atome d'azote et de phosphore en tant que site de liaison au centre iridium du complexe ;
Y¹, Y², Y³ et Y⁴, indépendamment les uns des autres, sont des atomes d'hydrogène, alkyle en C₁₋₁₂, cycloalkyle en C₅₋₁₀, ou un groupe aromatique ; ou au moins deux de ceux-ci forment conjointement un groupe ponté au moins divalent d'au moins 2 atomes de carbone ; et
Y^{⊝} est un anion, en particulier choisi dans le groupe constitué d'halogénure, PF₆⁻, SbF₆⁻, tétra(3,5-bis(trifluorométhyl)phényl)borate (BAr_{F}⁻), BF4⁻, des sulfonates perfluorés, de préférence F₃C-SO₃⁻ ou F₉C₄-SO₃⁻ ; ClO₄⁻, Al(OC₆F₅)₄⁻, Al(OC(CF₃)₃)₄⁻, N(SO₂CF₃)₂⁻, N(SO₂C₄F₉)₂⁻ et B(C₆F₅)₄⁻.

8. Procédé selon l'une quelconque des revendications précédentes 1 à 6 **caractérisé en ce que** le complexe d'iridium chiral dans les étapes c) et/ou f) est un complexe d'iridium chiral de formule (III) 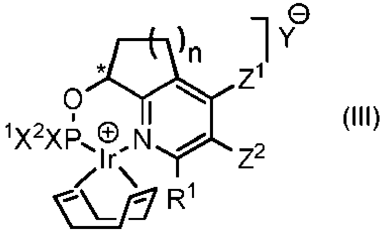 dans lequel
n est 1 ou 2 ou 3, de préférence 1 ou 2 ;
X¹ et X² sont, indépendamment l'un de l'autre, des atomes d'hydrogène, alkyle en C₁₋₄, cycloalkyle en C₅₋₇, adamantyle, phényle (facultativement substitué par un à trois groupes alkyle en C₁₋₅, alcoxy en C₁₋₄, perfluoroalkyle en C₁₋₄ et/ou un à cinq atomes d'halogène)), benzyle, 1-naphtyle, 2-naphtyle, 2-furyle ou ferrocényle ;
Z¹ et Z² sont, indépendamment l'un de l'autre, des atomes d'hydrogène, des groupes alkyle en C₁₋₅ ou alcoxy en C₁₋₅ ; ou Z¹ et Z² désignent conjointement un groupe de pontage formant un cycle de 5 ou 6 chaînons ;
Y^{⊝} est un anion, en particulier choisi dans le groupe constitué d'halogénure, PF₆⁻, SbF₆⁻, tétra(3,5-bis(trifluorométhyl)phényl)borate (BAr_{F}⁻), BF4⁻, des sulfonates perfluorés, de préférence F₃C-SO₃⁻ ou F₉C₄-SO₃⁻; ClO₄⁻, Al(OC₆F₅)₄⁻, Al(OC(CF₃)₃)₄⁻, N(SO₂CF₃)₂⁻, N(SO₂C₄F₉)₂⁻ et B(C₆F₅)₄⁻ ;
R¹ représente phényle ou o-tolyle ou m-tolyle ou p-tolyle ou un groupe de formule (IVa) ou (IVb) ou (IVc) 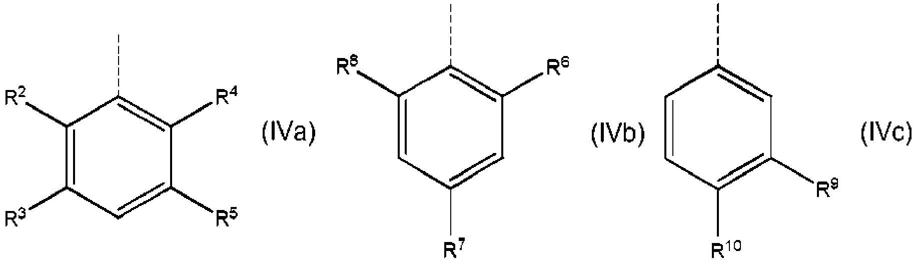 dans lequel R² et R³ représentent tous deux H ou un groupe alkyle en C₁-C₄ ou un groupe alkyle en C₁-C₄ halogéné ou représentent un groupe divalent formant conjointement un cycle cycloaliphatique ou aromatique à 6 chaînons qui est facultativement substitué par des atomes d'halogène ou par des groupes alkyle en C₁-C₄ ou par des groupes alcoxy en C₁-C₄
R⁴ et R⁵ représentent tous deux H ou un groupe alkyle en C₁-C₄ ou un groupe alkyle en C₁-C₄ halogéné ou un groupe divalent formant conjointement un cycle cycloaliphatique ou aromatique à 6 chaînons qui est facultativement substitué par des atomes d'halogène ou par des groupes alkyle en C₁-C₄ ou par des groupes alcoxy en C₁-C₄ ;
R⁶ et R⁷ et R⁸ représentent chacun un groupe alkyle en C₁-C₄ ou un groupe alkyle en C₁-C₄ halogéné ;
R⁹ et R¹⁰ représentent tous deux H ou un groupe alkyle en C₁-C₄ ou un groupe alkyle en C₁-C₄ halogéné ou un groupe divalent formant conjointement un cycle cycloaliphatique ou aromatique à 6 chaînons qui est facultativement substitué par des atomes d'halogène ou par des groupes alkyle en C₁-C₄ ou par des groupes alcoxy en C₁-C₄ ;
et dans lequel * représente a centre stéréogénique du complexe de formule (III).

9. Procédé selon la revendication 8 **caractérisé en ce que** le complexe d'iridium chiral de formule (III) utilisé dans l'étape c) et/ou f) utilisé pour l'hydrogénation asymétrique a la configuration S au centre stéréogénique indiqué par * dans le cas où la (3E,5E)- ou (3Z,5E)-6,10-diméthylundéca-3,5,9-trién-2-one ou des cétals de celles-ci, ou la (R,E)-6,10,14-triméthylpentadéc-5-én-2-one, ou un cétal de celle-ci, doivent être hydrogénés ;
ou a la configuration R au centre stéréogénique indiqué par * dans le cas où la (3E,5Z)- ou (3Z,5Z)-6,10-diméthylundéca-3,5,9-trién-2-one ou des cétals de celles-ci, ou la (R,Z)-6,10,14-triméthylpentadéc-5-én-2-one, ou un cétal de celle-ci, doivent être hydrogénés.

10. Procédé de fabrication de (R,R)-isophytol ((3RS,7R,11R)-3,7,11,15-tétraméthylhexadéc-1-én-3-ol) comprenant
un procédé de fabrication de (6R,10R)-6,10,14-triméthylpentadécan-2-one comme décrit dans le procédé selon l'une quelconque des revendications précédentes 1 à 9 ; suivi par les étapes de soit
g) éthynylation de (6R,10R)-6,10,14-triméthylpentadécan-2-one en utilisant de l'éthyne en présence d'une substance basique pour obtenir le (7R,11R)-3,7,11,15-tétraméthylhexadéc-1-yn-3-ol ;
h) hydrogénation de (7R,11R)-3,7,11,15-tétraméthylhexadéc-1-yn-3-ol avec de l'hydrogène moléculaire en présence d'un catalyseur de Lindlar pour obtenir le (R,R)-isophytol ;
ou
h') vinylation de (6R,10R)-6,10,14-triméthylpentadécan-2-one par ajout d'un réactif de Grignard vinylique pour obtenir le (R,R)-isophytol.

11. Procédé de fabrication d'un composé de formule (V) comprenant
un procédé de fabrication de (6R,10R)-6,10,14-triméthylpentadécan-2-one comme décrit dans le procédé selon l'une quelconque des revendications précédentes 1 à 9 ; suivi par les étapes de
soit
g) éthynylation de (6R,10R)-6,10,14-triméthylpentadécan-2-one en utilisant de l'éthyne en présence d'une substance basique pour obtenir le (7R,11R)-3,7,11,15-tétraméthylhexadéc-1-yn-3-ol ;
h) hydrogénation du (7R,11R)-3,7,11,15-tétraméthylhexadéc-1-yn-3-ol avec de l'hydrogène moléculaire en présence d'un catalyseur de Lindlar pour obtenir le (R,R)-isophytol ;
ou
h') vinylation de (6R,10R)-6,10,14-triméthylpentadécan-2-one par ajout d'un réactif de Grignard vinylique pour obtenir le (R,R)-isophytol ;
suivie par les étapes de
m) condensation du (R,R)-isophytol avec un composé de formule (VI) pour obtenir un composé de formule (V) étant un mélange d'isomères compte tenu de la chiralité au centre indiqué par # ; 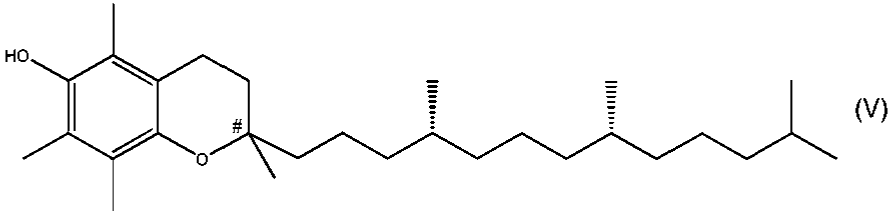 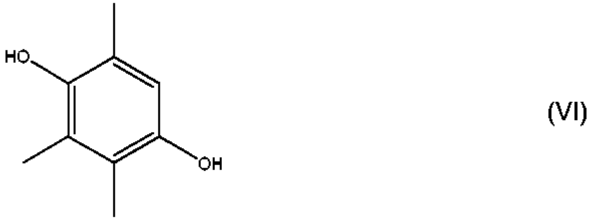
où # représente un centre stéréogénique.

12. Procédé de fabrication d'un composé de formule (V-A) comprenant
un procédé de fabrication de (6R,10R)-6,10,14-triméthylpentadécan-2-one comme décrit dans le procédé selon l'une quelconque des revendications précédentes 1 à 9 ;
suivi par les étapes de
soit
g) éthynylation de (6R,10R)-6,10,14-triméthylpentadécan-2-one en utilisant de l'éthyne en présence d'une substance basique pour obtenir le (7R,11R)-3,7,11,15-tétraméthylhexadéc-1-yn-3-ol ;
h) hydrogénation du (7R,11R)-3,7,11,15-tétraméthylhexadéc-1-yn-3-ol avec de l'hydrogène moléculaire en présence d'un catalyseur de Lindlar pour obtenir le (R,R)-isophytol ;
ou
h') vinylation de (6R,10R)-6,10,14-triméthylpentadécan-2-one par ajout d'un réactif de Grignard vinylique pour obtenir le (R,R)-isophytol ;
suivi par les étapes de
m) condensation de (R,R)-isophytol avec un composé de formule (VI) pour obtenir un composé de formule (V) étant un mélange d'isomères compte tenu de la chiralité au centre indiqué par # ; 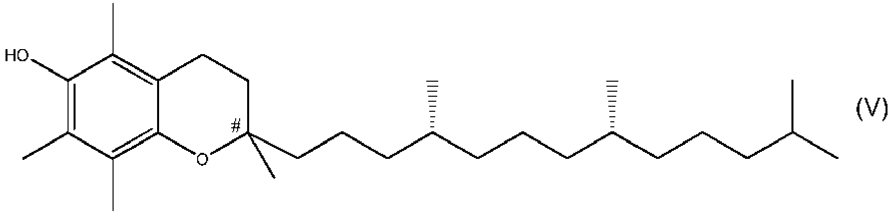 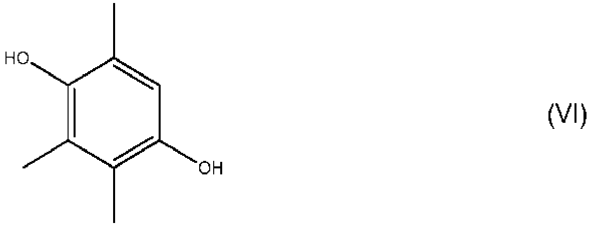 où # représente un centre stéréogénique ;
et
n) isolement d'un composé de formule (V-A) à partir du mélange d'isomères de formule (V)

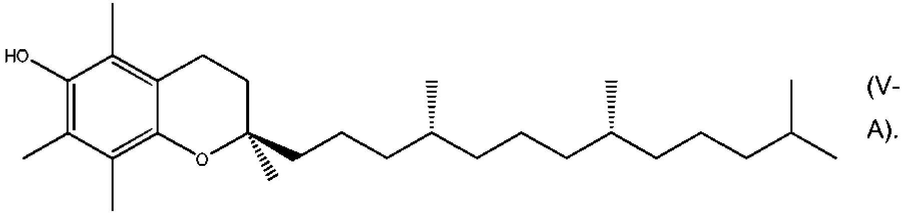

13. Composition comprenant
- au moins un cétal de formule (XI) et
- au moins un complexe d'iridium chiral 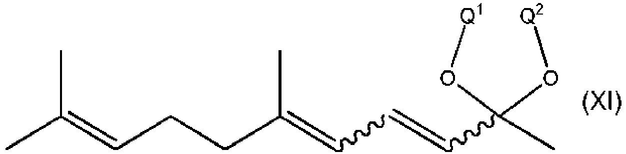 dans lequel un trait ondulé représente une liaison carbone-carbone qui est liée à la double liaison carbone-carbone adjacente de sorte que ladite double liaison carbone-carbone soit dans la configuration Z ou E ;
et dans lequel
Q¹ et Q² désignent individuellement ou tous deux un groupe alkyle en C₁-C₁₀ ou un groupe alkyle en C₁-C₁₀ halogéné ; ou forment conjointement un groupe alkylène en C₂-C₆ ou un groupe cycloalkylène en C₆-C₈.

14. Cétal qui est choisi dans le groupe constitué du (R)-6,10-diméthyl-2,2-bis(2,2,2-trifluoroéthoxy)undécane, du 2,5,5-triméthyl-2-((4R,8R)-4,8,12-triméthyltridécyl)-1,3-dioxane et du (6R,10R)-6,10,14-triméthyl-2,2-bis(2,2,2-trifluoroéthoxy)pentadécane.
